# EUROPEAN PATENT APPLICATION

(11) **EP 4 726 044 A1**
(43) Date of publication of application: **15.04.2026**
(21) Application number: 24383092.4
(22) Date of filing: 08.10.2024
(51) Int. Cl.: C12N 15/115, G01N 33/569

(54) **HIV APTAMERS AND USES THEREOF**

(71) Applicant: Fundación para la Investigación Biomédica del Hospital Universitario Ramón y Cajal, 28034 Madrid (ES)
(72) Inventor: HOLGUÍN FERNÁNDEZ, África, 28034 Madrid (ES); VALADÉS ALCARAZ, Ana, 28034 Madrid (ES); REINOSA FERNÁNDEZ, Roberto, 28034 Madrid (ES)
(74) Representative: Hoffmann Eitle

(57) **Abstract**

In the present invention, there is provided a nucleic acid molecule that is capable of recognizing and/or binding to HIV, in particular to the integrase (IN) of HIV-1 and HIV-2 variants or portions thereof. In particular, the nucleic acid molecule is capable of binding to the HCINp1 (11 aa) (SEQ ID NO 4, PYNPQSQGWE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKWPRRK) and/or HCINp5 (10aa) (SEQ ID NO 15, LLWKGEGAW). The nucleic acid molecule may be a polynucleotide or an oligonucleotide.

## Description

### Technical field

The invention lies in the field of HIV aptamers, HIV aptamer biosensors, and methods of their use.

### Background art.

HIV, a lentivirus of the *Retroviridae* family, is responsible for the global AIDS pandemic. By 2023, around 40 million people lived with HIV, with 1.3 million new infections annually. Current diagnostic methods, including serologic and molecular tests, have limitations in early HIV detection. Serologic tests identify anti-HIV antibodies around 21 days post-infection and not earlier, and are inadequate for newborns of HIV-positive mothers due to persistent maternal antibodies, resulting in potential false-positive results for up to 18 months. The WHO recommends nucleic acid amplification techniques (NAATs) to detect the viral genome as early as 10 days post-infection, but they are costly in high-prevalence regions. This underscores the need for affordable molecular tests for rapid point-of-care (POC) detection. Currently, POC rapid antibody tests and NAATs are the primary diagnostic tools, but alternative methods targeting HIV proteins (more abundant than 2 HIV genome copies per virion) could improve early detection and HIV confirmation.

Fourth- and fifth-generation immunoassays detect HIV antibodies and P24 capsid protein 2-3 weeks after exposure, and they are not confirmatory. The most sensitive require expensive infrastructure and are not POC assays, such as Bioplex 2200 HIV Ag-Ab (Biorad), the HIV-1 P24 ELISA Kit (Abcam) and the Simoa HIV P24 Advantage Kit (Quanterix) detecting 5.2 pg/mL, 1.1 pg/mL and 0.00024 pg/mL of P24 antigen, respectively. Rapid POC serological tests for HIV screening such as Determine^{™} HIV Early Detect (Abbott) offer faster and less expensive P24 detection compared to ELISA. However, detecting ultra-low levels of P24 and other HIV proteins in the first week of infection remains challenging. HIV integrase (IN) is a 288 amino acid viral crucial for integrating proviral DNA into the host genome, a key step in viral replication and transmission. IN forms a 66 kDa homodimer, with approximately 250 IN molecules per virus, compared to the two copies of viral RNA.

Developing innovative, highly sensitive molecular diagnostic systems for the early detection of viral proteins and HIV confirmation in pediatric and adult populations worldwide could be promising, potentially offering more accurate methods than those based solely on viral genome.

Aptamer technology represents a breakthrough approach to diagnostics, utilizing single-stranded RNA or DNA molecules that acquire three-dimensional folds that enable them to bind molecular targets, including viral proteins, with high affinity. Aptamers offer several advantages over monoclonal antibodies, including shorter production times, reduced costs and improved stability with longer storage at room temperature. Aptasensors (aptamers coupled to nanoparticles or biosensors) have been developed for the detection of several viral proteins, including NS1 of Dengue and Zika viruses, E6 of HPV16, GP of Ebola virus, HCV core protein, HBeAg, S1 of SARS-CoV-2 and HA of Influenza A virus.

HIV is subdivided into 2 types (HIV-1 and HIV-2). HIV-1, causing 99% infections worldwide, includes four groups (M, O, N, and P), with group M comprising 10 subtypes, at least 157 circulating recombinant forms (CRFs), and numerous unique recombinant forms (URFs). The high viral mutation rate and recombination events lead to considerable genetic variability, which can affect the precision of NAAT primers, probes, and assays. While newer immunoassays have improved the detection of acute infections, ongoing evaluation is needed. The present invention identifies exposed integrase (IN) peptides highly-conserved across HIV variants and aptamers that recognize these viral peptides with high affinity and sensitivity for their future integration in ultrasensitive biosensors.

### Brief description of the figures

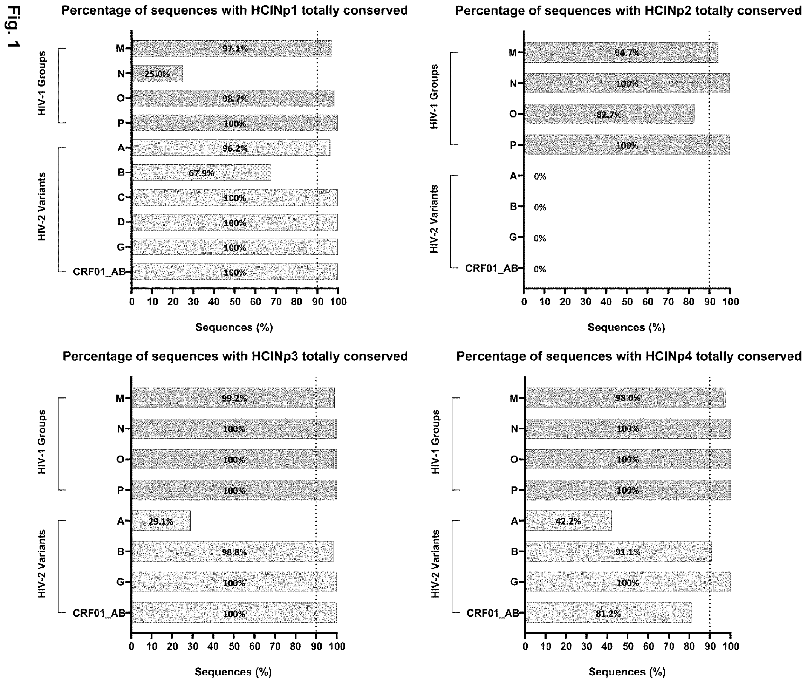

**Figure 1****. Percentage of LANL IN sequences containing peptides HCINp1, HCINp2, HCINp3,** and **HCINp4** in **HIV-1** and **HIV-2.** The dotted line indicates 90% conservation across analysed IN sequences. HIV-2 variants E, F, H, and I lacked sufficient sequences for the conservation analysis of all peptides. CRF, Circulating Recombinant Forms.

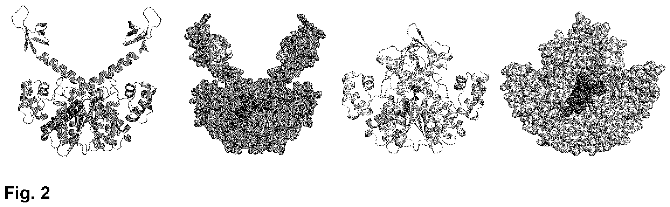

**Figure 2****. Locations of peptides HCINp1, HCINp2, HCINp3 and HCINp4 in HIV-1 and HIV-2 integrases 3D structures.** The 3D structures of HIV-1 and HIV-2 integrases were generated by AlphaFold 3, using the HXB2 reference sequence (GenBank NC_001802.1) for HIV-1 and the ALI reference sequence (GenBank AF082339.1) for HIV-2, and were displayed by The PyMOL Molecular Graphics System v2.5.2 program. The proteins are shown as cartoon and spheres with the following colour code: teal, HIV-1 integrase; pink, HIV-2 integrase. HCINp1 is coloured in red, HCINp2 in orange, HCINp3 in yellow and HCINp4 in green in both HIV integrases.

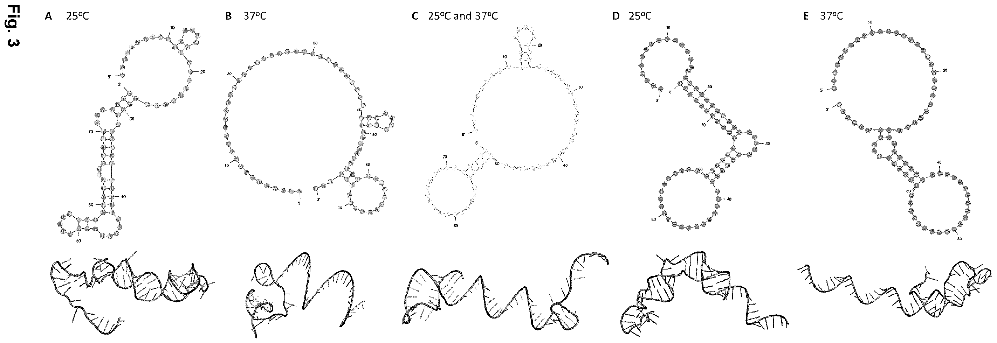

**Figure 3****. Predicted secondary and 3D structures of selected anti-IN aptamers (Apt1-HCINp1, Apt2-HCINp1 and Apt3-HCINp1**) using the DNA Folding bioinformatic tool. Predicted secondary and 3D structures of: Apt1-HCINp1 at 25°C (A) and at 37°C (B); Apt2-HCINp1 at both 25°C and 37°C (C); Apt3-HCINp1 at 25°C (D), and at 37°C (E).

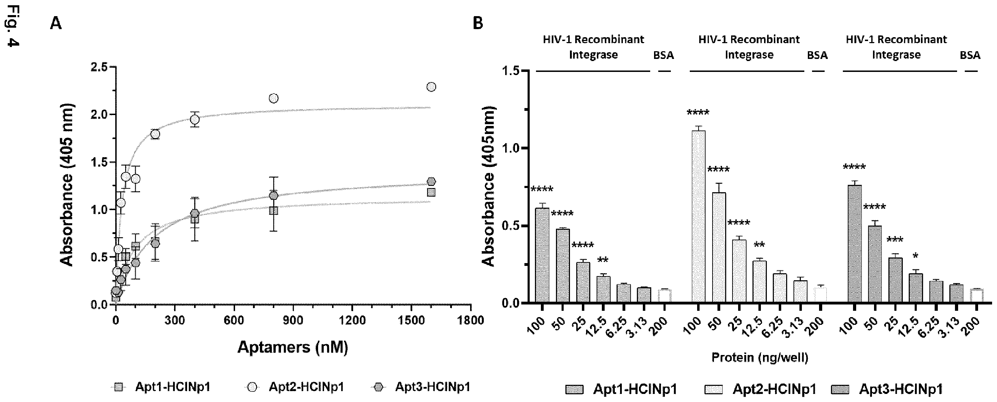

**Figure 4****. Binding capability or affinity (A) and sensitivity (B) of anti-IN aptamers (Apt1-HCINp1, Apt2-HCINp1 and Apt3-HCINp1) for HIV-1 recombinant integrase (ARP-9420, BEI Resources) detection by indirect ELONA.** (A) The recombinant protein was plated at 100 ng/well and incubated with several concentrations (6.25-1,600 nM) of digoxigenin-labelled aptamers. (B) In all ELONA assays 200 ng of BSA were plated as negative control. The recombinant protein was plated to different concentrations (3.13-100 ng/well) and incubated with five times K_{D} of digoxigenin-labelled aptamers (463 nM, 155 nM and 982 nM of Apt1-HCINp1, Apt2-HCINp1 and Apt3-HCINp1, respectively). Significant p-values: *, p<0.05; **, p<0.01; ***, p<0.001; ****, p<0.0001. BSA, bovine serum albumin.

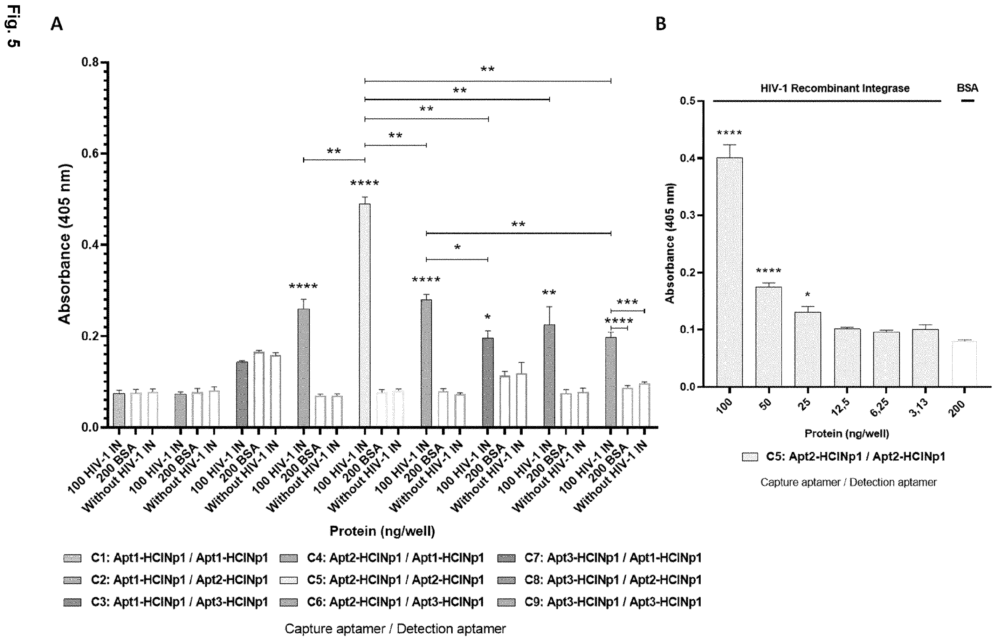

**Figure 5****. Capture/detection anti-IN aptamer combinations (C1-C9) (A) and sensitivity of the best one (C5) (B) for HIV-1 recombinant integrase recognition by sandwich ELONA.** Negative controls: 200 ng bovine serum albumin (A and B); and PBS/MgCl₂ without recombinant HIV-1 IN protein (A). Significant p-values: *, p<0.05; **, p<0.01; ***, p<0.001; ****, p<0.0001. (A) The capture biotin-labelled aptamer was plated at 0.5 µM and was incubated with 100 ng/well of HIV-1 recombinant IN and five times K_{D} of digoxigenin-labelled aptamers (463 nM, 155 nM and 982 nM of Apt1-HCINp1, Apt2-HCINp1 and Apt3-HCINp1, respectively). (B) The capture biotin-labelled aptamer was plated at 0.5 µM and was incubated with different concentrations (3.13-100 ng/well) of HIV-1 recombinant IN or 200 ng of BSA as negative control, and five times K_{D} of digoxigenin-labelled aptamer (155 nM of Apt2-HCINp1). IN, HIV recombinant integrase (ARP-9420, BEI Resources); BSA, bovine serum albumin.

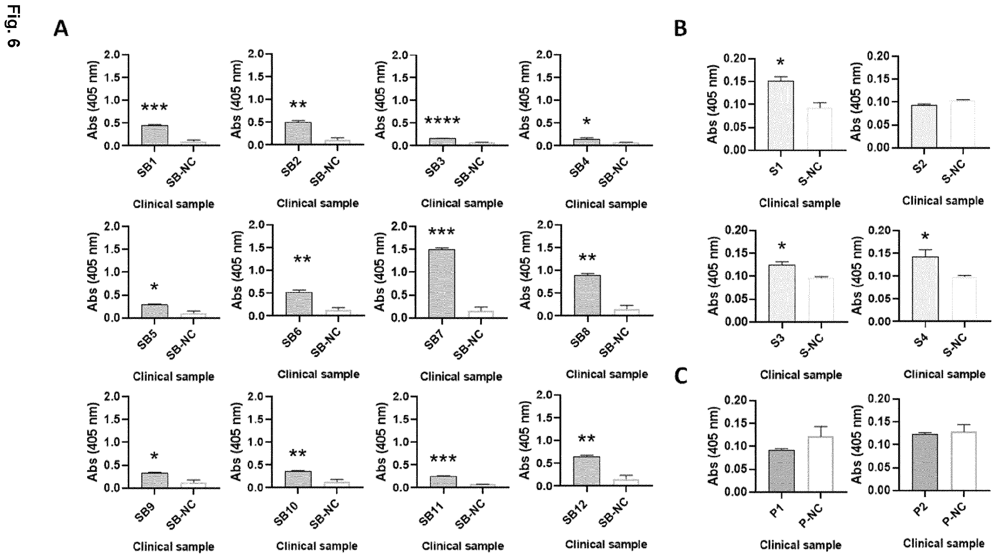

**Figure 6****. HIV-1 IN detection in HIV-infected free-cells viral culture supernatants (A), sera (B), and plasma (C) using C5, the best anti-IN aptamer combination (Apt2-HCINp1 as capture and detection aptamer).** Negative controls: SB-NC (PBS/MgCl₂), S-NC (HIV-uninfected serum), and P-NC (HIV-uninfected plasma). Significant P values: *, p<0.05; **, p<0.01; ***, p<0.001; ****, p<0.0001. The capture biotin-labelled aptamer was plated at 0.5 µM and was incubated with 100 µL/well of each clinical sample (SB, S and P) and five times K_{D} of digoxigenin-labelled aptamer (155 nM of Apt2-HCINp1). Specific p-values vs. the corresponding negative controls: For viral supernatants: SB1 (A), p = 0.0005; SB2 (A), p = 0.0022; SB3 (B), p < 0.0001; SB4 (B), p = 0.0433; SB5 (C), p = 0.0194; SB6 (C), p = 0.0068; SB7 (D), p = 0.0001; SB8 (D), p = 0.0011; SB9 (CRF01_AE), p = 0.0257; SB10 (CRF01_AE), p = 0.0159; SB11 (CRF02_AG), p = 0.0003; and SB12 (CRF02_AG), p = 0.0057. For serum: S1, p = 0.0139; S2, p>0.05; S3, p = 0.0203; S4, p = 0.0450). Abs, absorbance; SB, viral supernatant; S, serum; P, plasma; NC, negative control.

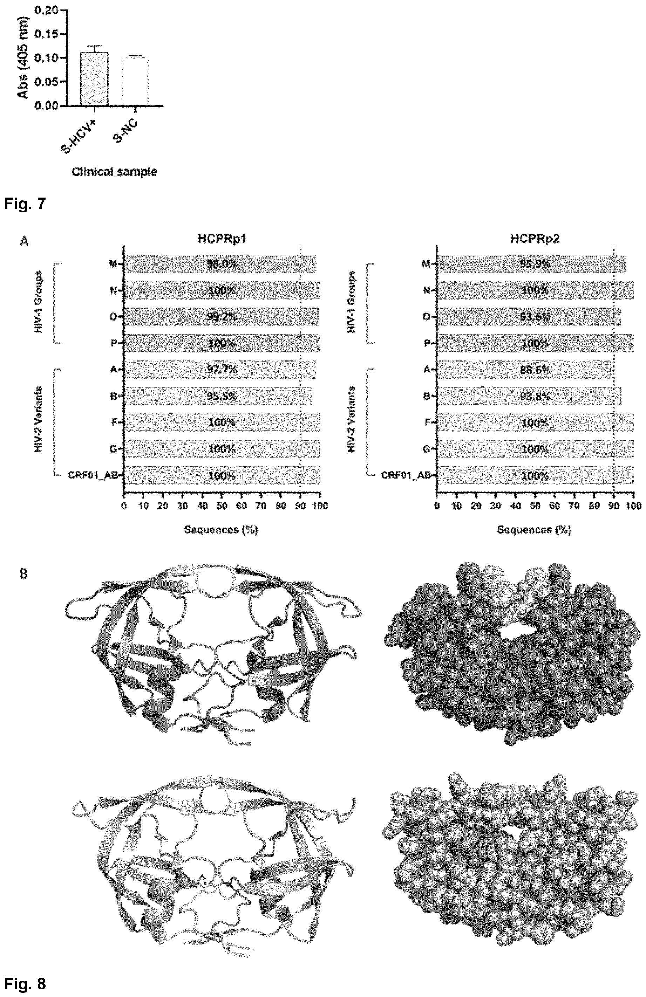

**Figure 7****. Absence of HCV recognition in an HCV-infected serum using C5, the best anti-IN aptamer combination (Apt2-HCINp1 as capture and detection aptamer).** S-NC (both HIV and HCV uninfected serum). The capture biotin-labelled aptamer was plated at 0.5 µM and was incubated with 100 µL/well of serum and five times K_{D} of digoxigenin-labelled aptamer (155 nM of Apt2-HCINp1). No significant difference (p=0.2435). Abs, absorbance; S, serum; NC, negative control.
**Figure 8****. Percentage of LANL sequences with peptides HCPRp1 and HCPRp2 (A) and their location in HIV-1 and HIV-2 proteases 3D structures (B).** (A) Dotted line at 90% of sequences with the peptide totally conserved across analysed sequences. Number of sequences analysed of each HIV-1 group for HCPRp1: M, 216,793; N, 10; O, 265; P, 2, and each HIV-2 variant: A, 1,340; B, 88; F, 1; G, 1; CRF01_AB, 15. Number of sequences analysed of each HIV-1 group for HCPRp2: M, 219,163; N, 11; O, 297; P, 1, and each HIV-2 variant: A, 1,424; B, 97; F, 1; G, 1; CRF01_AB, 15. HIV-2 variants C, D, E, H, and I had no sequences available for the conservation analysis of both peptides. CRF, circulating recombinant forms. (B) The crystallographic structures were obtained from RCSB PDB (2P3A, HIV-1 protease; 3S45, HIV-2 protease) and were displayed by The PyMOL Molecular Graphics System v2.5.2 program. The proteins are shown as cartoon (left) and spheres (right) with the following colour code: teal, HIV-1 protease; pink, HIV-2 protease. HCPRp1 is coloured in orange and HCPRp2 in yellow in both HIV proteases.

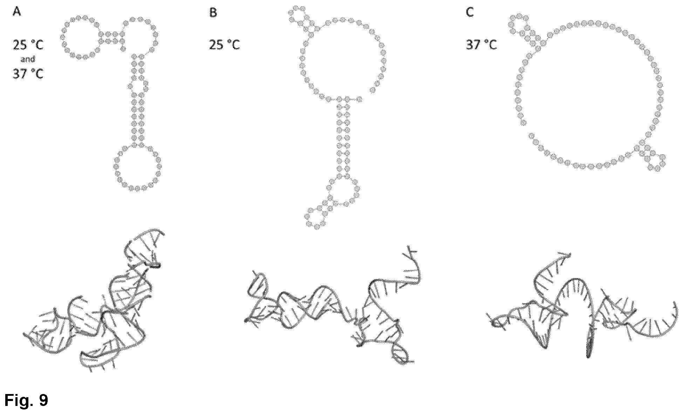

**Figure 9****. Predicted secondary and 3D structure of selected Apt1-HCPRp2 (A) and Apt2-HCPRp2 (B and C) anti-PR aptamers.** The DNA sequences of Apt1-HCPRp2 and Apt2-HCPRp2 were analysed using DNA Folding Form Application. (A) The predicted secondary and 3D structure of Apt1-HCPRp2 was the same at 25 °C and 37 °C. (B) Secondary and 3D structure of Apt2-HCPRp2 at 25 °C and (C) 37 °C.

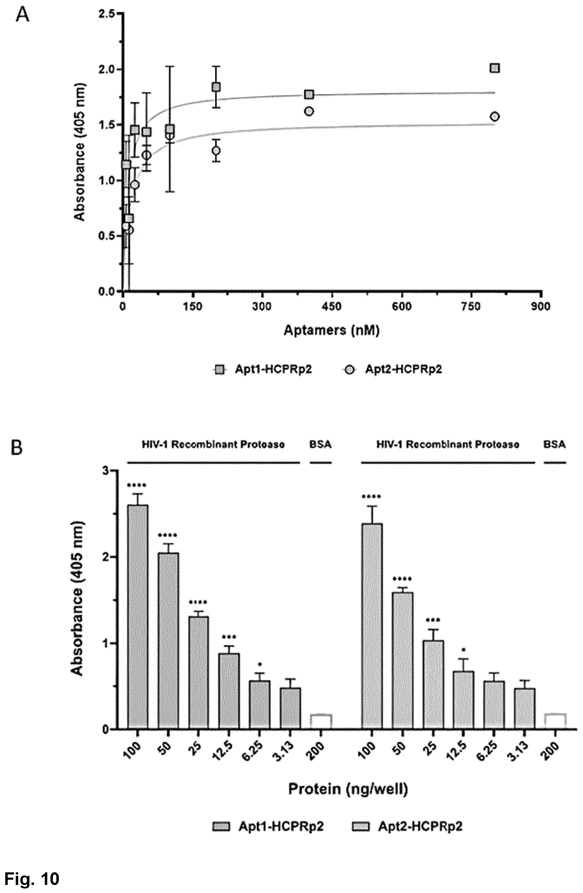

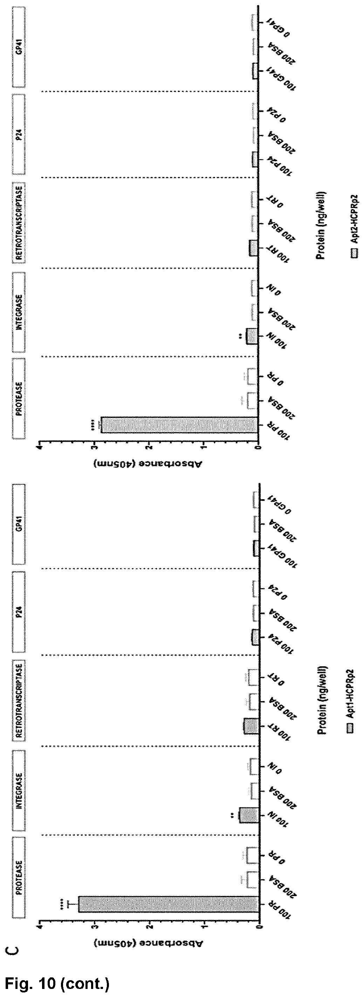

**Figure 10****. Binding capability (A), sensitivity (B), and specificity (C) of Apt1-HCPRp2 (left) and Apt2-HCPRp2 (right) for HIV-1 recombinant protease (ARP-11781, BEI Resources) by ELONA.** In all ELONA assays 200 ng of BSA were plated as negative controls. (A) The recombinant protein was plated at 100 ng/well and incubated with several concentrations (6.25-800 nM) of digoxigenin-labelled aptamers. (B) The recombinant protein was plated to different concentrations (3.13-100 ng/well) and incubated with five times K_{D} of digoxigenin-labelled aptamers (50-75 nM of Apt1-HCPRp2 and Apt2-HCPRp2, respectively). (C) In all ELONA assays, 100 ng of HIV recombinant proteins (PR, RT, IN, P24, or Gp41) were plated per well in 96-well microtiter plates and were incubated with five times K_{D} of digoxigenin-labelled aptamer Apt1-HCPRp2 (50 nM) (left) or Apt2-HCPRp2 (75 mM) (right). We also included 200 ng/well of BSA and 0 ng/well of proteins as negative controls. BSA, bovine serum albumin.

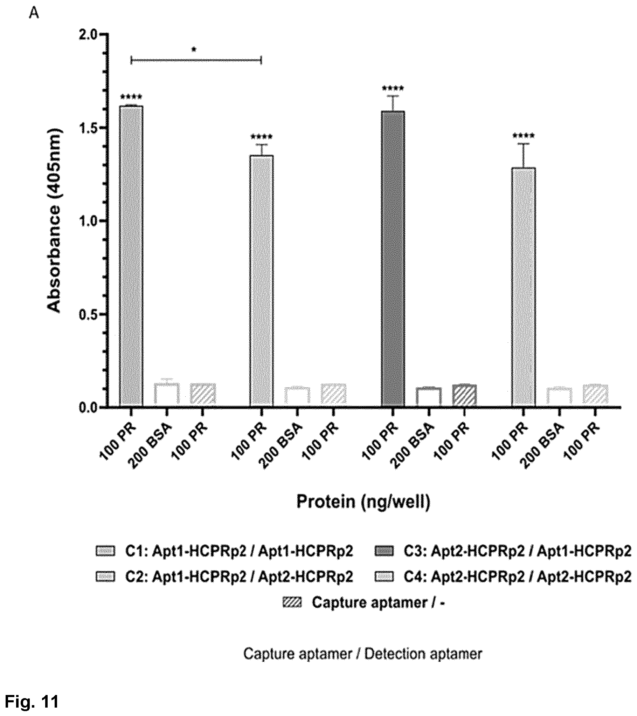

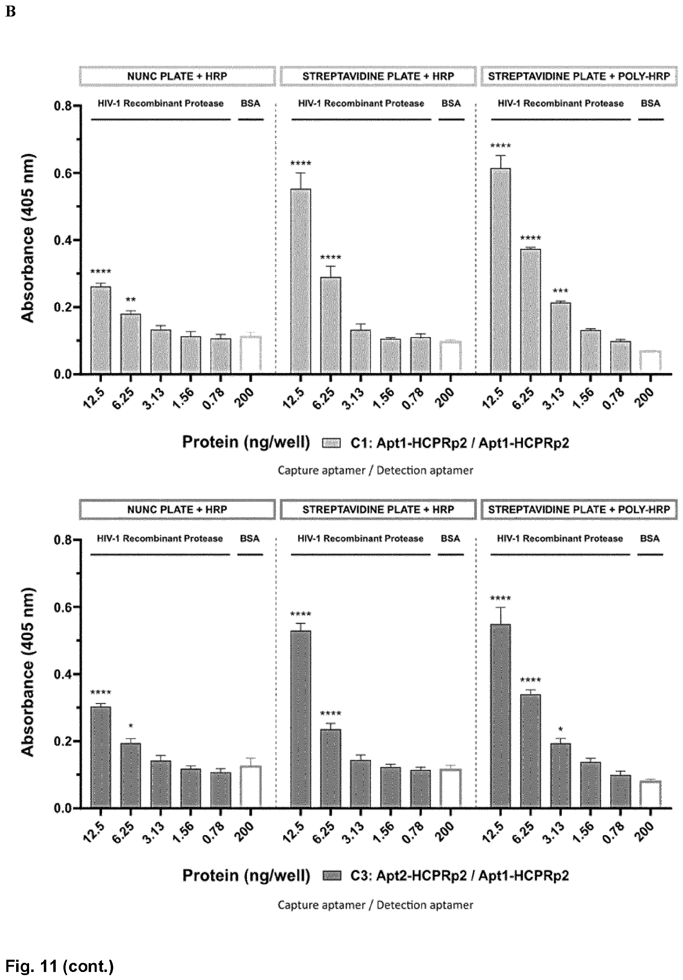

**Figure 11****. Best capture/detection aptamer combinations (A) and sensitivity (B) of C1 and C3 for HIV-1 recombinant protease (ARP-11781, BEI Resources) by sandwich ELONA.** (A) The capture aptamer was plated at 0.5 µM and was incubated with 100 ng/well of HIV-1 recombinant protease and five times K_{D} of digoxigenin-labelled aptamers (50-75 nM of Apt1-HCPRp2 and Apt2-HCPRp2, respectively). (B) The capture aptamer was plated at 0.5 µM and was incubated with different concentrations (0.78-12.5 ng/well) of HIV-1 recombinant protease or 200 ng of BSA as negative control (NC), and five times K_{D} of digoxigenin labelled aptamer Apt1-HCPRp2 [50 nM]. PR, HIV recombinant protease; BSA, bovine serum albumin; HRP, horseradish peroxidase.

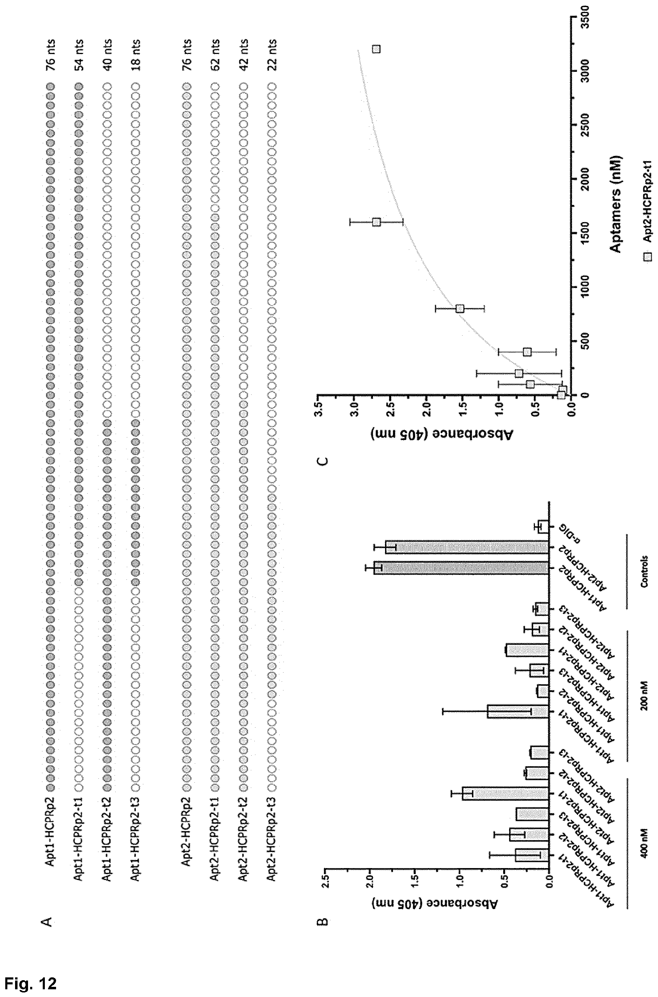

**Figure 12****. Apt-HCPRp2 truncated aptamers (A), detection capacity of HIV-1 recombinant protease compared to their parental aptamers (B) and affinity of Apt2-HCPRp2-t1 (C).** (A) Coloured circles represent the ssDNA aptamers sequence nucleotides and non-coloured circles represents the truncated nucleotides for each aptamer vs. their parental aptamer. Nts, nucleotides. (B) The HIV-1 recombinant protein was plated at 100 ng/well and incubated with two concentrations (400 - 200 nM) of digoxigenin labelled truncated aptamers. For the positive controls, the concentration of digoxigenin-labelled aptamers were five times their K_{D} (50-75 nM of Apt1-HCPRp2 and Apt2-HCPRp2, respectively), and for the negative control, the concentration of secondary anti-digoxigenin-HRP antibody was 1:1000 without digoxigenin-labelled aptamers (C) The HIV-1 recombinant protein was plated at 100 ng/well and incubated with several concentrations (0-3,200 nM) of digoxigenin-labelled truncated aptamer.

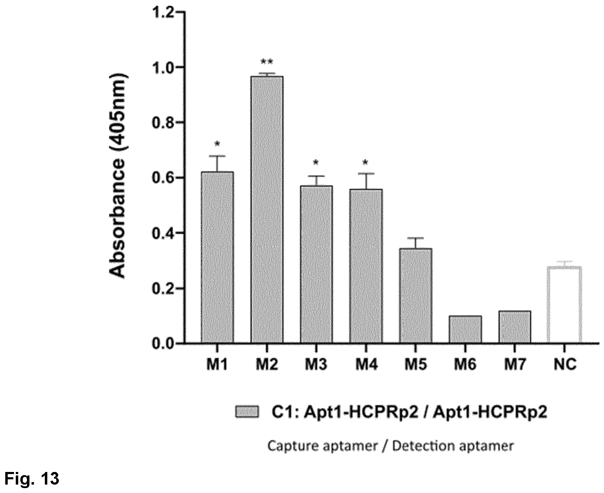

**Figure 13****. Detection of HIV infected samples with C1, the best combination of aptamers able to detect PR.** The capture aptamer labelled with biotin was plated at 0.5 µM in a 96-well microtiter plate coated with streptavidin. After the samples pre-treatment previously explained in Materials and Methods, the plate was incubated with 100 µL/well of the different samples (three wells/sample) and five times K_{D} of digoxigenin-labelled detection aptamer [50 nM]. We used anti-digoxigenin-poly-HRP as secondary detection antibody. NC, negative control (negative HIV DBS or dried blood sample). M6 and M7 were tested in only one sandwich ELONA.

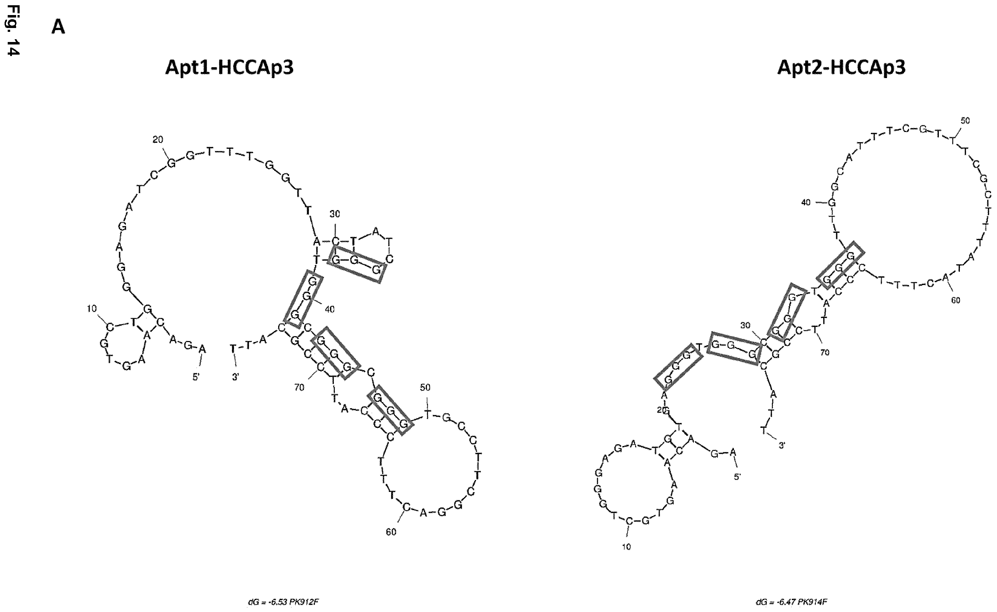

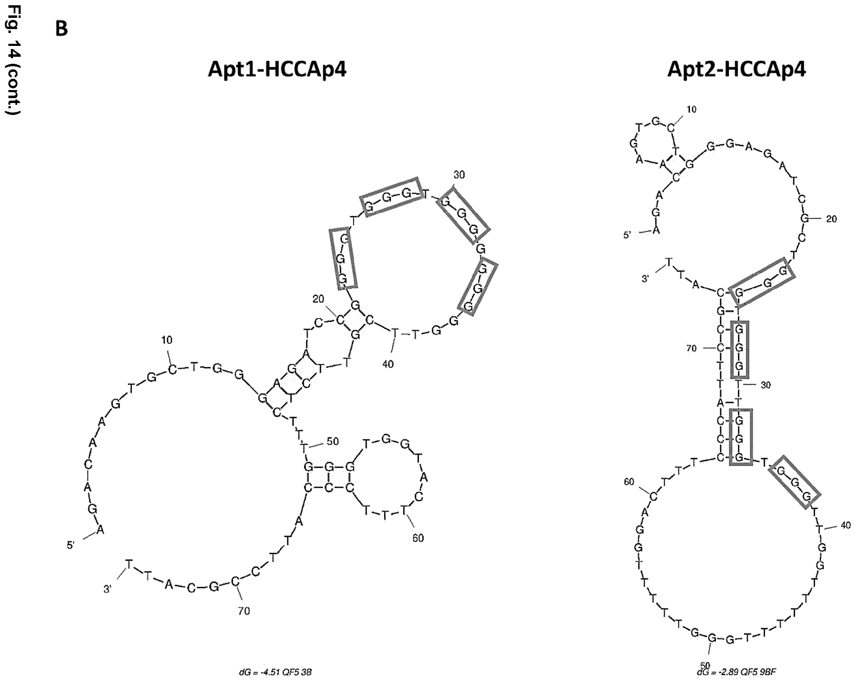

**Figure 14****. Predicted secondary structures of selected anti-P24 aptamers Apt1-HCCAp3, Apt2-HCCAp3 (A), Apt1-HCCAp4 and Apt2-HCCAp4 (B).** The DNA sequences were analysed using the DNA Folding Form Application.

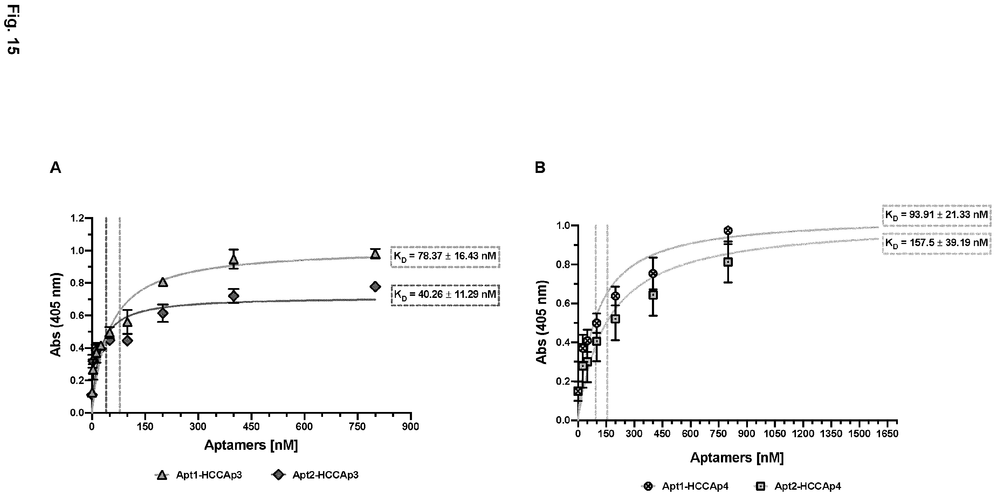

**Figure 15****. Binding capability or affinity of Apt1-HCCAp3, Apt2-HCCAp3 (A), Apt1-HCCAp4 and Apt2-HCCAp4 (B) for HIV-1 recombinant P24 (ARP-13126, BEI Resources) detection by indirect ELONA.** The recombinant protein was plated at 100 ng/well and incubated with several concentrations (minimum 3.13 nM to maximum 1,600 nM) of digoxigenin-labelled aptamers. Abs, absorbance.

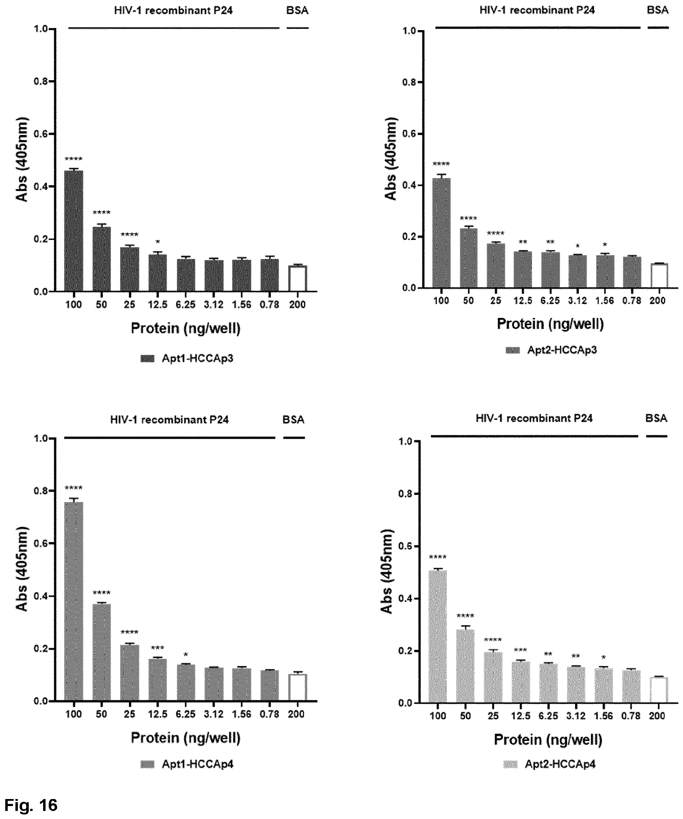

**Figure 16****. Sensitivity of anti-P24 aptamers (Apt1-HCCAp3, Apt2-HCCAp3, Apt1-HCCAp4 and Apt2-HCCAp4) for HIV-1 recombinant P24 (ARP-13126, BEI Resources) by indirect ELONA.** In all ELONA assays 200 ng of BSA were plated as negative control. The recombinant protein was plated to different concentrations (0.78-100 ng/well) and incubated with five times K_{D} of digoxigenin-labelled aptamers (392 nM, 201 nM, 368.1 nM, 787.5 nM of Apt1-HCCAp3, Apt2-HCCAp3, Apt1-HCCAp4, Apt2-HCCAp4, respectively). Significant p-values: *, p<0.05; **, p<0.01; ***, p<0.001; ****, p<0.0001. Abs, absorbance; BSA, bovine serum albumin.

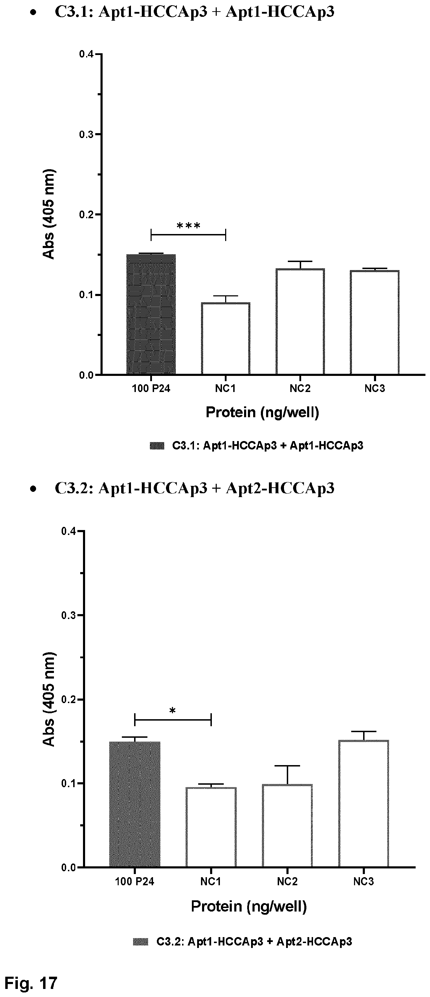

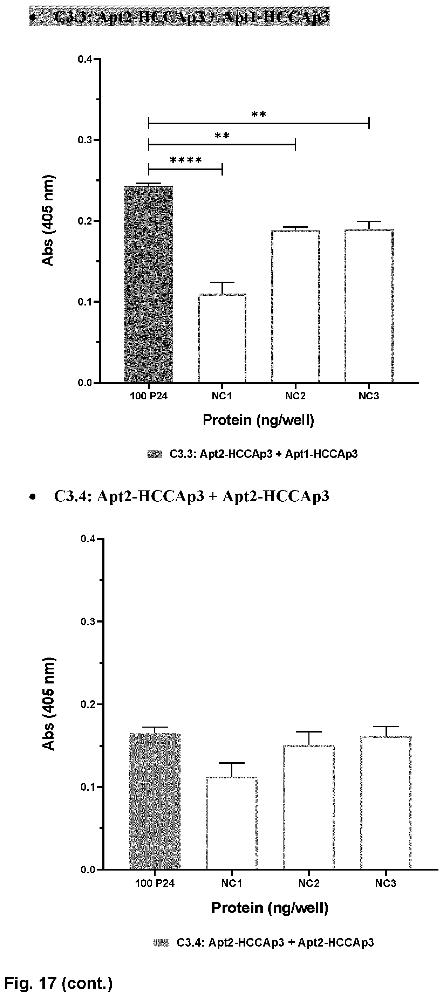

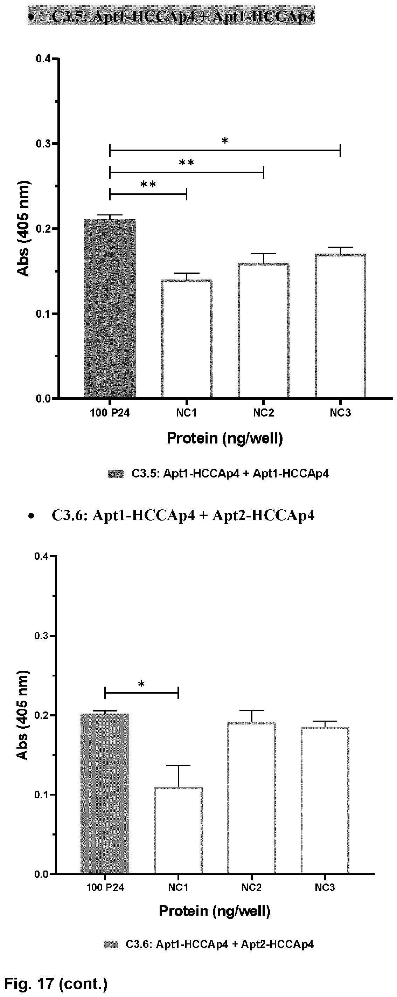

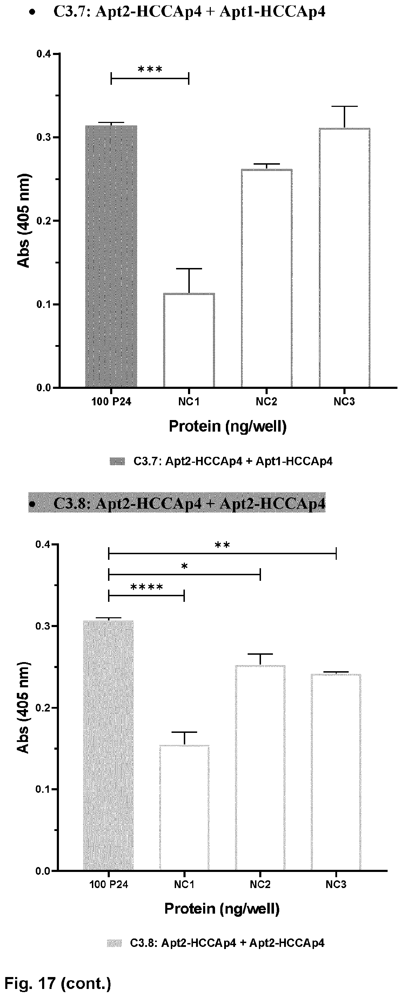

**Figure 17****. Best capture/detection combinations of anti-P24 aptamers (C3.1-C3.8) directed against the same peptide (HCCAp3 or HCCAp4) for HIV-1 recombinant P24 (ARP-13126, BEI Resources) by sandwich ELONA.** Negative controls: HIV-1 recombinant P24 without detection aptamer (NC1), 200 ng of BSA (NC2) and PBS/MgCl₂ without recombinant protein (NC3). The non-labelled aptamer was plated at 0.5 µM and was incubated with 100 ng/well of HIV-1 recombinant P24 and five times K_{D} of digoxigenin-labelled aptamers (392nM, 201 nM, 368.1 nM and 787.5 nM of Apt1-HCCAp3, Apt2-HCCAp3, Apt1-HCCAp4 and Apt2-HCCAp4, respectively). Significant p-values: *, p<0.05; **, p<0.01; ***, p<0.001; ****, p<0.0001. Abs, absorbance; P24, HIV-1 capsid protein; BSA, bovine serum albumin.

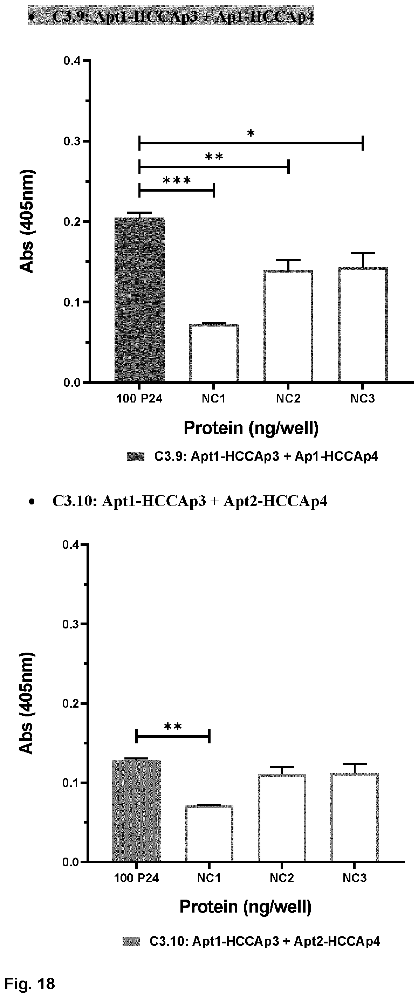

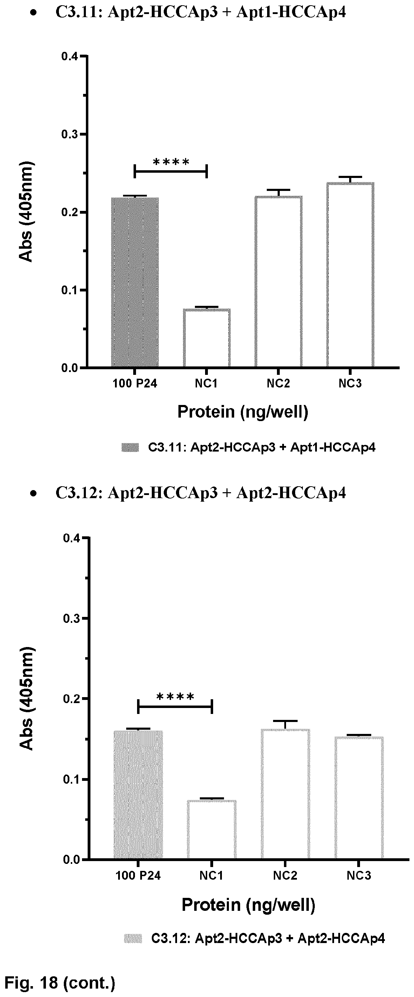

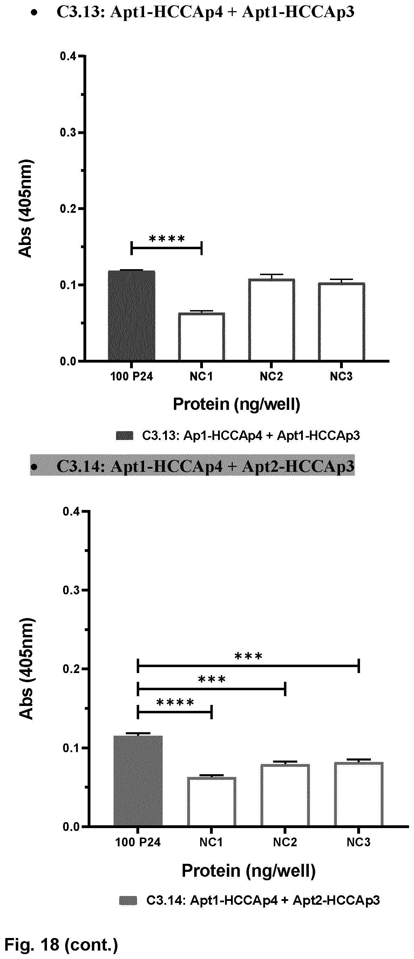

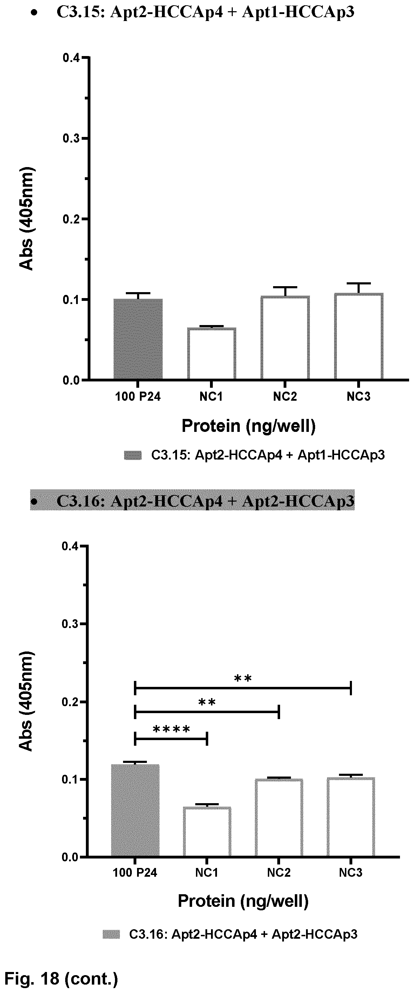

**Figure 18****. Best capture/detection combinations of anti-P24 aptamers (C3.9-C3.16) directed against different peptides (HCCAp3 and HCCAp4) for HIV-1 recombinant P24 (ARP-13126, BEI Resources) by sandwich ELONA.** Negative controls: HIV-1 recombinant P24 without detection aptamer (NC1), 200 ng of BSA (NC2) and PBS/MgCl₂ without recombinant protein (NC3). The capture biotin-labelled aptamer was plated at 0.5 µM and was incubated with 100 ng/well of HIV-1 recombinant P24 and five times K_{D} of digoxigenin-labelled aptamers (392nM, 201 nM, 368.1 nM and 787.5 nM of Apt1-HCCAp3, Apt2-HCCAp3, Apt1-HCCAp4 and Apt2-HCCAp4, respectively). Significant p-values: *, p<0.05; **, p<0.01; ***, p<0.001; ****, p<0.0001. Abs, absorbance; P24, HIV-1 capsid protein, BSA, bovine serum albumin.

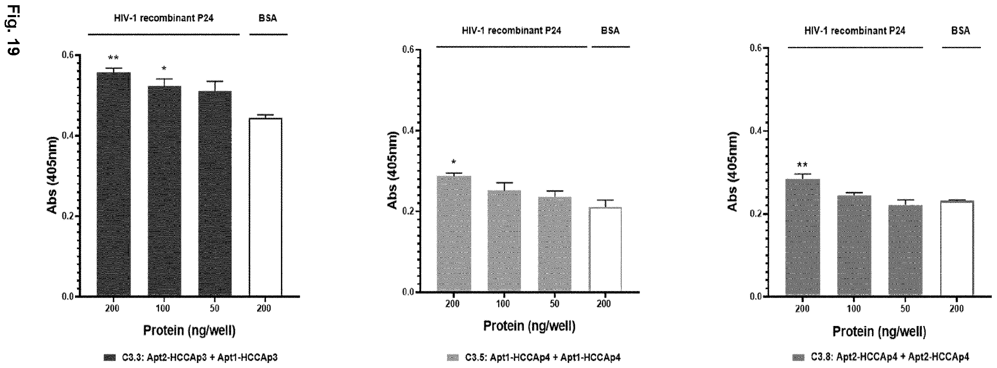

**Figure 19****. Sensitivity of the best capture/detection combinations of anti-P24 aptamers (C3.3, C3.5, C3.8) directed against the same peptide (HCCAp3 or HCCAp4) for HIV-1 recombinant P24 (ARP-13126, BEI Resources) by sandwich ELONA.** The capture non-labelled aptamer was plated at 0.5 µM and was incubated with different concentrations (50-200 ng/well) of HIV-1 recombinant P24 or 200 ng of BSA as negative control, and five times K_{D} of digoxigenin-labelled aptamer (392 nM, 368.1 nM, 787.5 nM of Ap1-HCCAp3, Apt1-HCCAp4 and Apt2-HCCAp4, respectively). Significant p-values: *, p<0.05; **, p<0.01. Abs, absorbance; P24, HIV-1 capsid protein; BSA, bovine serum albumin.

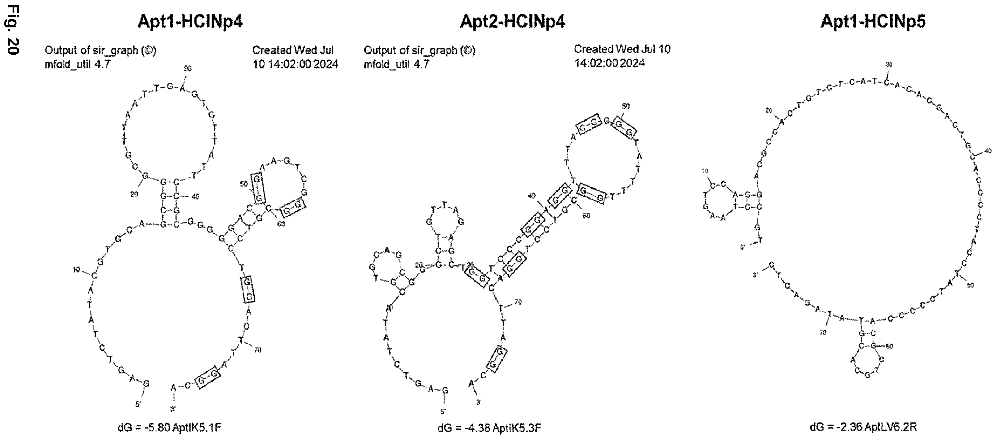

**Figure 20****. Predicted secondary structures of new anti-IN aptamers (Apt1-HCINp4, Ap2-HCINp4 and Apt1-HCINp5).** The DNA sequences were analysed using the DNA Folding Form Application.

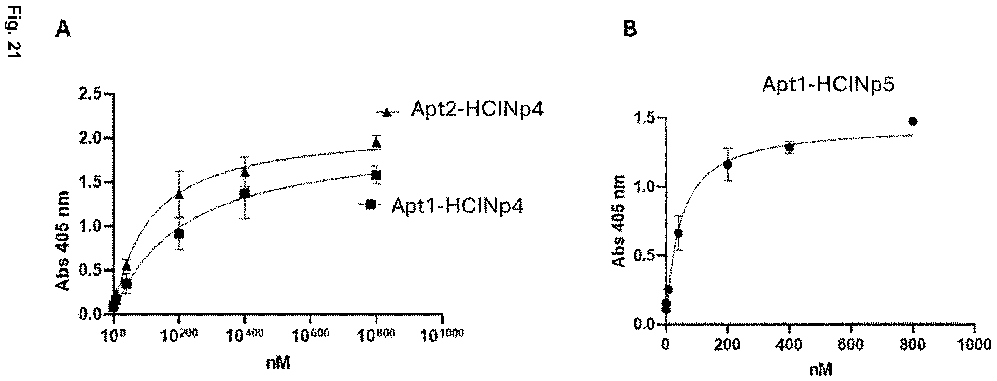

**Figure 21****. Binding capability or affinity of Apt1-HCINp4, Apt2-HCINp4 (A) and Apt1-HCINp5 (B) for HIV-1 recombinant integrase (HRP-20203, BEI Resources) detection by indirect ELONA.** The recombinant protein was plated at 100 ng/well and incubated with several concentrations (3.13-800 nM) of digoxigenin-labelled aptamers. Abs, absorbance.

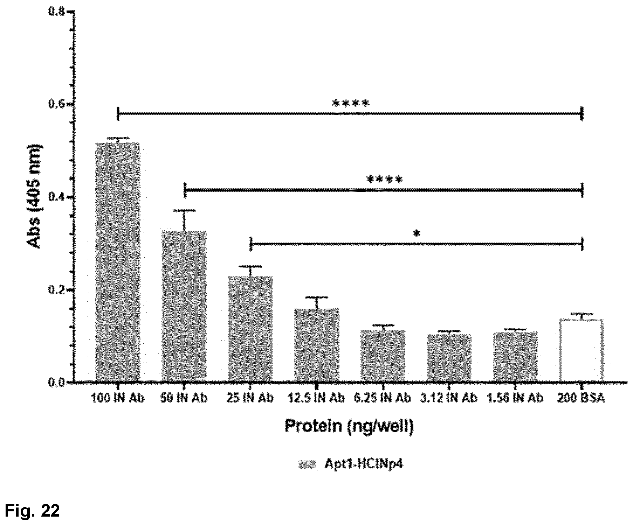

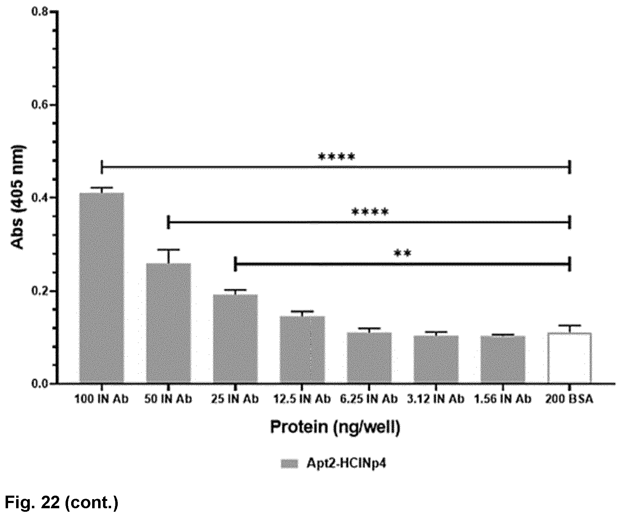

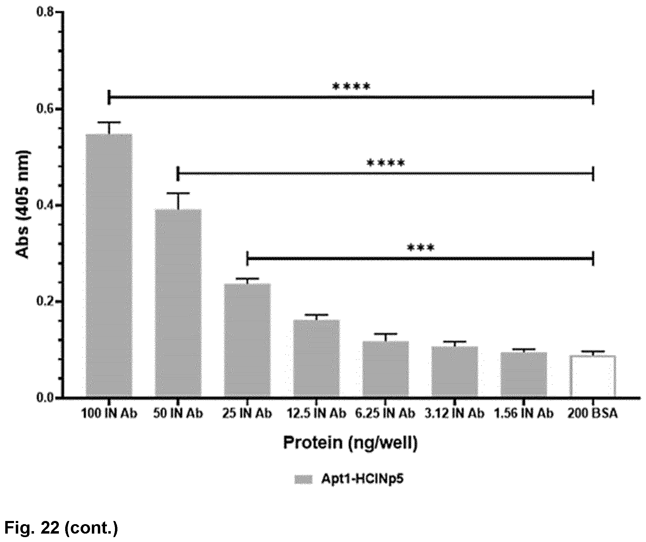

**Figure 22****. Sensitivity of new anti-IN aptamers (Apt1-HCINp4, Apt2-HCINp4 and Apt1-HC!Np5) for HIV-1 recombinant integrase by indirect ELONA.** In all ELONA assays 200 ng of BSA were plated as negative control. The recombinant protein was plated to different concentrations (1.56-100 ng/well) and incubated with five times K_{D} of digoxigenin-labelled aptamers (1000 nM, 543.5 nM, and 222.45 nM of Apt1-HCINp4, Apt2-HCINp4 and Apt1-HCINp5, respectively). Significant p-values: *, p<0.05; **, p<0.01; ***, p<0.001; ****, p<0.0001. Abs, absorbance; IN Ab, HIV-1 recombinant integrase (A58225, Antibodies); BSA, bovine serum albumin.

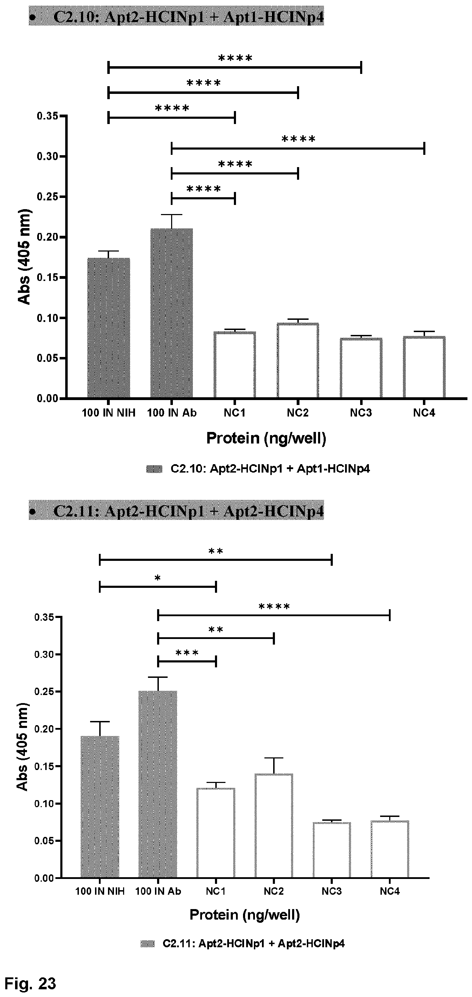

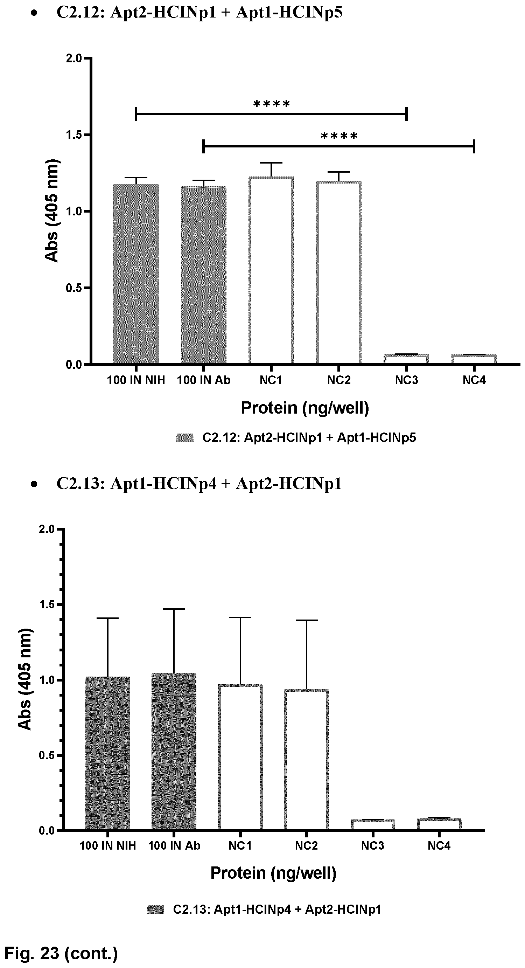

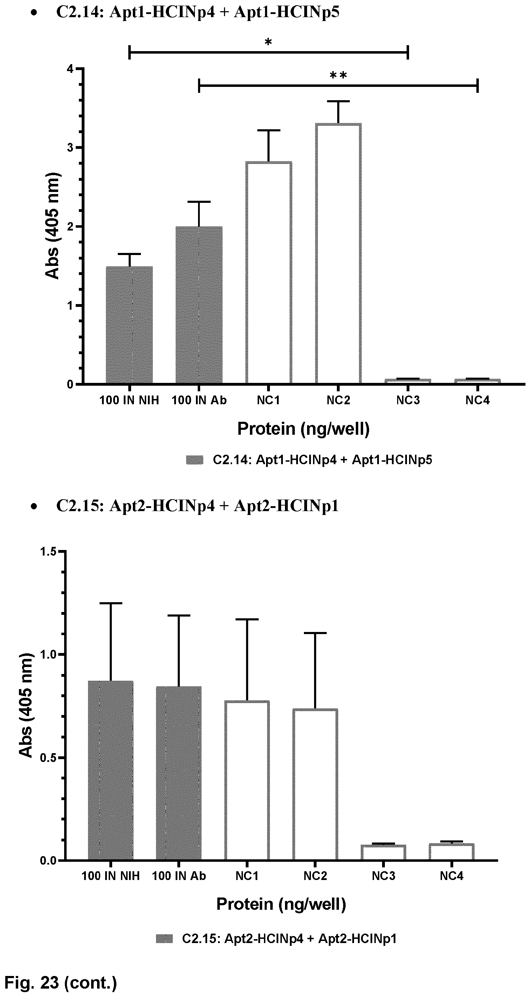

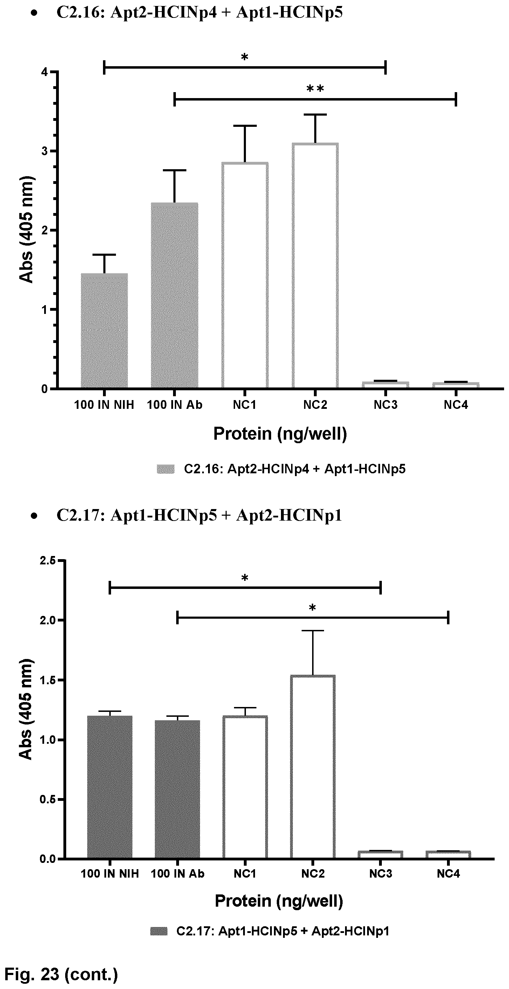

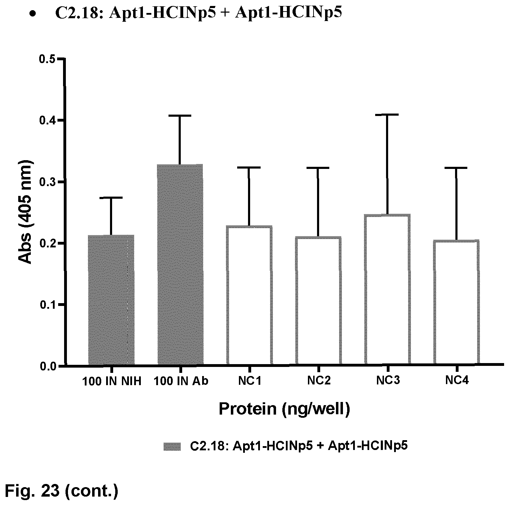

**Figure 23****. Best new capture/detection combinations of anti-IN aptamers (C2.10** - **C2.18) for HIV-1 recombinant integrase recognition by sandwich ELONA.** Negative controls: 200 ng of BSA (NC1), PBS/MgCl₂ without recombinant protein (NC2), IN NIH without detection aptamer (NC3) and IN Ab without detection aptamer (NC4). The capture biotin-labelled aptamer was plated at 0.5 µM and was incubated with 100 ng/well of HIV-1 recombinant IN NIH or IN Ab and five times K_{D} of digoxigenin-labelled aptamers (155 nM, 1000 nM, 543.5 nM, and 222.45 nM of Apt2-HCINp1, Apt1-HCINp4, Apt2-HCINp4, and Apt1-HCINp5, respectively). Significant p-values: *, p<0.05; **, p<0.01; ***, p<0.001; ****, p<0.0001. Abs, absorbance; IN NIH, HIV-1 recombinant integrase (HRP-20203, BEI Resources); IN Ab, HIV-1 recombinant integrase (A58225, Antibodies); BSA, bovine serum albumin.

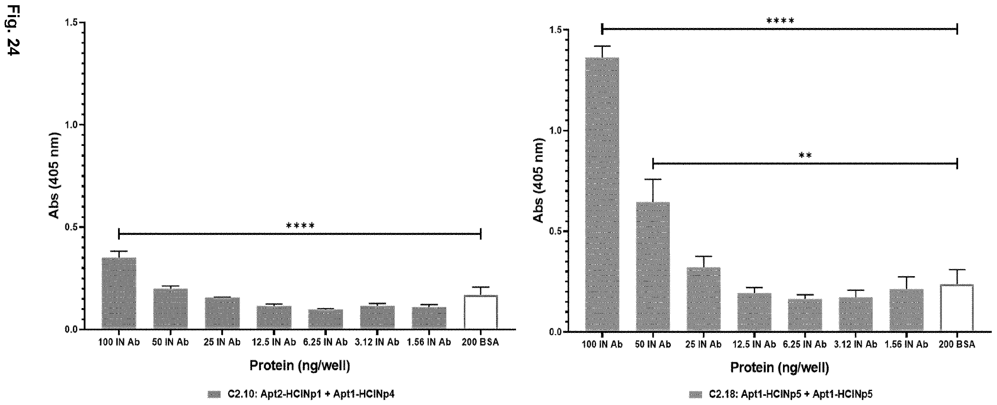

**Figure 24****. Sensitivity of two new capture/detection combinations of anti-IN aptamers (C2.10, C2.18) for HIV-1 recombinant integrase by sandwich ELONA.** The capture biotin-labelled aptamer was plated at 0.5 µM and was incubated with different concentrations (1.56-100 ng/well) of HIV-1 recombinant IN or 200 ng of BSA as negative control, and five times K_{D} of digoxigenin-labelled aptamer (1000 nM, 222.45 nM of Apt1-HCINp4 and Apt1-HCINp5, respectively). Significant p-values: **, p<0.01; ****, p<0.0001. Abs, absorbance; IN Ab, HIV-1 recombinant integrase (A58225, Antibodies); BSA, bovine serum albumin.

### DEFINITIONS

The term "aptamer" as used herein broadly refers to a nucleic acid molecule that is capable of binding with high affinity and specificity to a target molecule, in particular to a HIV virus protein. An "aptamer" not only includes nucleic acid molecules composed of natural bases, but also include those comprising unnatural or artificial bases, modified bases and/or nucleic acid analogs. An "aptamer" may also have modifications. Non-limiting examples of such modifications include: terminus modification to increase a stability of the molecule, functional group modification (e.g. amino, thiol, ethyl, diol), conjugation modification (e.g. biotinylation) and phosphorothioate bond modification.

The term "identifying" as used herein in relation to an infection is to be interpreted broadly to encompass determining a presence, an absence, an amount, a level of disease burden, a phase or a nature of the infection.

The term "treatment", "treat" and "therapy", and synonyms thereof as used herein refer to both therapeutic treatment and prophylactic or preventative measures, wherein the object is to prevent or slow down (lessen) a medical condition, which includes but is not limited to diseases (such as HIV infection), symptoms and disorders. A medical condition also includes a body's response to a disease or disorder, e.g. inflammation. Those in need of such treatment include those already with a medical condition as well as those prone to getting the medical condition or those in whom a medical condition is to be prevented.

The term "subject" as used herein includes patients. The term "patient" refers to individuals suffering or are likely to suffer from a medical condition such as a HIV virus infection.

The term "particle" as used herein broadly refers to a discrete entity or a discrete body. The particle described herein can include an organic, an inorganic or a biological particle. The particle used described herein may also be a macro particle that is formed by an aggregate of a plurality of sub-particles or a fragment of a small object. The particle of the present disclosure may be spherical, substantially spherical, or non-spherical, such as irregularly shaped particles or ellipsoidally shaped particles. The term "size" when used to refer to the particle broadly refers to the largest dimension of the particle. For example, when the particle is substantially spherical, the term "size" can refer to the diameter of the particle; or when the particle is substantially non-spherical, the term "size" can refer to the largest length of the particle.

The terms "coupled" or "connected" as used in this description are intended to cover both directly connected or connected through one or more intermediate means, unless otherwise stated.

The term "associated with", used herein when referring to two elements refers to a broad relationship between the two elements. The relationship includes, but is not limited to a physical, a chemical or a biological relationship. For example, when element A is associated with element B, elements A and B may be directly or indirectly attached to each other or element A may contain element B or vice versa.

The term "adjacent" used herein when referring to two elements refers to one element being in close proximity to another element and may be but is not limited to the elements contacting each other or may further include the elements being separated by one or more further elements disposed therebetween. The term "and/or", e.g., "X and/or Y" is understood to mean either "X and Y" or "X or Y" and should be taken to provide explicit support for both meanings or for either meaning.

Further, in the description herein, the word "substantially" whenever used is understood to include, but not restricted to, "entirely" or "completely" and the like. In addition, terms such as "comprising", "comprise", and the like whenever used, are intended to be non-restricting descriptive language in that they broadly include elements/components recited after such terms, in addition to other components not explicitly recited. For example, when "comprising" is used, reference to a "one" feature is also intended to be a reference to "at least one" of that feature. Terms such as "consisting", "consist", and the like, may in the appropriate context, be considered as a subset of terms such as "comprising", "comprise", and the like. Therefore, in embodiments disclosed herein using the terms such as "comprising", "comprise", and the like, it will be appreciated that these embodiments provide teaching for corresponding embodiments using terms such as "consisting", "consist", and the like. Further, terms such as "about", "approximately" and the like whenever used, typically means a reasonable variation, for example a variation of +/- 5% of the disclosed value, or a variance of 4% of the disclosed value, or a variance of 3% of the disclosed value, a variance of 2% of the disclosed value or a variance of 1 % of the disclosed value.

Furthermore, in the description herein, certain values may be disclosed in a range. The values showing the end points of a range are intended to illustrate a preferred range. Whenever a range has been described, it is intended that the range covers and teaches all possible sub-ranges as well as individual numerical values within that range. That is, the end points of a range should not be interpreted as inflexible limitations. For example, a description of a range of 1 % to 5% is intended to have specifically disclosed sub-ranges 1 % to 2%, 1 % to 3%, 1 % to 4%, 2% to 3% etc., as well as individually, values within that range such as 1 %, 2%, 3%, 4% and 5%. The intention of the above specific disclosure is applicable to any depth/breadth of a range.

Additionally, when describing some embodiments, the disclosure may have disclosed a method and/or process as a particular sequence of steps. However, unless otherwise required, it will be appreciated that the method or process should not be limited to the particular sequence of steps disclosed. Other sequences of steps may be possible. The particular order of the steps disclosed herein should not be construed as undue limitations. Unless otherwise required, a method and/or process disclosed herein should not be limited to the steps being carried out in the order written. The sequence of steps may be varied and still remain within the scope of the disclosure.

Furthermore, it will be appreciated that while the present disclosure provides embodiments having one or more of the features/characteristics discussed herein, one or more of these features/characteristics may also be disclaimed in other alternative embodiments and the present disclosure provides support for such disclaimers and these associated alternative embodiments.

### DESCRIPTION OF EMBODIMENTS

Current studies on HIV aptamers are primarily focused on their application as therapeutic agents, with emerging research exploring their diagnostic potential. HIV Integrase protein can be a diagnostic target, and not only therapeutic target. Aptamers targeting conserved regions of HIV proteins show promise for molecular detection. As shown in the examples of the present specification, with further refinement and more advanced detection nanotechnology-based systems than the ELONA assay, sensitivity levels comparable or ideally superior to 4th and 5th generation immunoassays and to NAATs could be achieved. Utilizing nanotechnology, these aptamers could be integrated into point-of-care devices for earlier HIV diagnosis, regardless of the patient's age or infection variant. Therefore, exemplary, non-limiting embodiments of a nucleic acid molecule, optionally an aptamer for HIV virus (HIV), and related methods, mixtures kits are disclosed hereinafter.

In various embodiments, there is provided a nucleic acid molecule that is capable of recognizing and/or binding to HIV, in particular to
- the integrase (IN) of HIV-1 and HIV-2 variants or portions thereof. In particular, the nucleic acid molecule is capable of binding to the HCINp1 (11 aa) (SEQ ID NO 4, PYNPQSQGVVE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKVVPRRK) and/or HCINp5 (10aa) (SEQ ID NO 15, LLWKGEGAVV);
   - the protease (PR) of HIV-1 and HIV-2 variants or portions thereof. In particular, the nucleic acid molecule is capable of binding to the HCPRp1: LLDTGADD (8aa) and/or HCPRp2: GGIGGFI (7 aa); or
   - the p24 capsid protein of HIV-1 and HIV-2 variants or portions thereof. In particular, the nucleic acid molecule is capable of binding to the HCCAp1 = PS peptide: PRGSDIAGTTS (11 aa, residues 99-109 P24), HCCAp2 = TC peptide: TLLVQNANPDC (11 aa, residues 188-198 P24), HCCAp3 = PK peptide: PRTLNAWVK (9 aa, residues 17-25 P24), HCCAp4 = QF peptide: QGPKEPFRDYVDRF (14 aa, residues 155-168 P24), HCCAp5 = KT peptide: KAFSPEVIPMFSALSEGATPQDLNT (25 aa, residues 30-54 P24) and/or HCCAp5 = KC peptide: KFGAEVVPGFQALSEGCTPYDINQMLNC (28 aa, residues 29-57 P26).

Preferably, the nucleic acid molecule is capable of binding to the HCINp1 (11 aa) (SEQ ID NO 4, PYNPQSQGWE).

The nucleic acid molecule may be a polynucleotide or an oligonucleotide. The nucleic acid molecule may be composed of deoxyribonucleic acid (DNA) or ribonucleic acid (RNA). The nucleic acid molecule may comprise natural bases, unnatural or artificial bases, modified bases and/or nucleic acid analogs (e.g., containing base analogs, sugar analogs and/or a non-native backbone and the like). A nucleic acid molecule may also have modifications, for example, at its terminus or termini. The nucleic acid molecule may be single-stranded. In various embodiments, the nucleic acid molecule is capable of binding to HIV or portions thereof with high affinity and/or high specificity. No particular size is implied by the term"nucleic acid molecule". In various embodiments, the nucleic acid molecule comprises an aptamer. In various embodiments therefore, there is provided an aptamer for HIV. The aptamer may be an RNA-based aptamer, or an aptamer comprising ribonucleotide units or it may be a DNA-based aptamer, or an aptamer comprising deoxyribonucleotide units. In one embodiment, the aptamer comprises a DNA- based aptamer. Advantageously, a DNA-based aptamer may have increased stability. A DNA-based aptamer includes one with modification(s).

In one embodiment, the aptamer is capable of binding specifically to a single type of HIV selected from the group consisting of type 1 or type 2. More preferably, the aptamer is capable of binding specifically to HIV-1, more particularly to any of the four groups; M, N, O and P. Preferably, to group M and more preferably to any of the distinct subtypes; A, B, C, D, F, G, H, J, K and L.

In various embodiments, the aptamer may comprise natural bases and/or unnatural bases (or artificial bases). A "natural base" refers to a naturally occurring base such as adenine (A), guanine (G), cytosine (C), thymine (T) (for a DNA-based aptamer) and uracil (U) (for an RNA-based aptamer). An "unnatural base" (or "artificial base") refers to a base which is not a naturally occurring base.

The aptamer may comprise a hairpin structure or a stem-loop structure. The aptamer may further comprise a bulge. In various embodiments, the aptamer comprises at least one hairpin structure or stem-loop structure and/or at least one bulge. In various embodiments, the unnatural base(s) resides in a loop structure of the aptamer. In various embodiments, the unnatural base(s) resides in a bulge of the aptamer. The bulge may or may not be in the loop structure. In various embodiments, the unnatural base(s) resides in a loop structure and/or a bulge of the aptamer. In some embodiments, from about one to about ten, or from about one to about five unnatural base(s) reside in a loop structure and/or a bulge of the aptamer. In some embodiments, at least about one, at least about two, at least about three, at least about four or at least about five unnatural base(s) resides in a loop structure and/or a bulge of the aptamer. In some embodiments, no more than about one, no more than about two, no more than about three, no more than about four or no more than about five unnatural base(s) resides in a loop structure and/or a bulge of the aptamer. In some embodiments, about one, about two, about three, about four, about five or more unnatural base(s) resides in a loop structure and/or a bulge of the aptamer. In some embodiments, all of the unnatural base(s) resides in a loop structure and/or a bulge of the aptamer. In some embodiments, a stem structure of the aptamer is devoid of an unnatural base. In some embodiments, the stem structure of the aptamer consists only of natural bases. In some embodiments, the loop structure and/or a bulge of the aptamer comprises unnatural base(s). In some embodiments, the unnatural base in the aptamer does not have a binding partner. In some embodiments, the unnatural base in the aptamer does not form a base pair with a natural base. In some embodiments, the unnatural base forms a looped/buldged out region in the aptamer. A bulge may therefore be a portion of a nucleic acid or aptamer that has not been paired. Non-pairing may arise due to non-complementarity/mismatch base-pairing among natural bases, non-complementarity/mismatch base-pairing among unnatural bases, or non-complementarity/mismatch base-pairing among natural base(s) and unnatural base(s). In some examples, non-pairing arises due to the introduction of one or more unnatural base (e.g. in a region of natural bases) that does not form a base pair with natural bases. In some examples, a bulge includes from about 1 to about 20 bases, from about 1 to about 15 bases, from about 1 to about 10 bases or from about 1 to about 5 bases. In some examples, a bulge includes about 1 , about 2, about 3, about 4, about 5, about 6, about 7, about 8, about 9, about 10, about 1 1 , about 12, about 13, about 14, about 15, about 16, about 17, about 18, about 19 or about 20 bases.

In various embodiments, the number of bases in the aptamer is from about 20 to about 200, from about 20 to about 100, from about 30 to about 100, from about 40 to about 100, from about 40 to about 90, from about 40 to about 80, from about 40 to about 70 or from about 50 to about 60. Advantageously, embodiments of the aptamer having a short length or small size may have reduced unexpected interactions/toxicity, reduced cost of material/production and improved material quality assurance.

In various embodiments, the unnatural base is selected from the group consisting of: 7-(2thienyl)imidazo[4,5-b]pyridine (Ds); 7-(2,2'-bithien-5- yl)imidazo[4,5-b]pyridin-3-yl group (Dss); pyrrole2-carbaldehyde (Pa); diol- modified pyrrole2-carbaldehyde (diol-Pa); 2-nitro-4-propynylpyrrole (Px); diol- modified 2-nitro-4-propynylpyrrole (diol-Px); 7-(2,2',5',2"-terthien-5- yl)imidazo[4,5-b]pyridin-3-yl group (Dsss); 2-amino-6-(2-thienyl)purin-9-yl group (s); 2-amino-6-(2,2'-bithien-5-yl)purin-9-yl group (ss); 2-amino-6-(2,2',5',2"- terthien-5-yl)purin-9-yl group (sss); 4-(2-thienyl)-pyrrolo[2,3-b]pyridin-1 -yl group (dDsa); 4-(2,2'-bithien-5-yl)-pyrrolo[2,3-b]pyridin-1 -yl group (Dsas); 4-[2-(2-thiazolyl)thien-5-yl]pyrrolo[2,3-b]pyridin-1 -yl group (Dsav); 4-(2-thiazolyl)- pyrrolo[2,3-b]pyridin-1 -yl group (dDva); 4-[5-(2-thienyl)thiazol-2-yl]pyrrolo[2,3- b]pyridin-1 -yl group (Dvas); 4-(2-imidazolyl)-pyrrolo[2,3-b]pyridin-1 -yl group (dDia); derivatives thereof and combinations thereof. In various embodiments, the unnatural base is selected from the group consisting of: 7- (2thienyl)imidazo[4,5-b]pyridine (Ds), 7-(2,2'-bithien-5-yl)imidazo[4,5-b]pyridin-3- yl group (Dss), pyrrole2-carbaldehyde (Pa), diol-modified pyrrole2-carbaldehyde (diol-Pa), 2-nitro-4-propynylpyrrole (Px), diol-modified 2-nitro-4-propynylpyrrole (diol-Px), derivatives thereof and combinations thereof. In some embodiments, the aptamer comprises an unnatural base consisting of Ds, diol-Pa, diol-Px and derivatives thereof. In some embodiments, the aptamer comprises an unnatural base consisting of Ds. In some embodiments, the aptamer comprises more Ds (or derivatives thereof) than diol-Pa (or derivatives thereof) and/or diol-Px (or derivatives thereof).

In various embodiments, the aptamer comprising at least one unnatural base is capable of recognizing and/or binding to HIV, in particular to
- the integrase (IN) of HIV-1 and HIV-2 variants or portions thereof. In particular, the aptamer is capable of binding to the HCINp1 (11 aa) (SEQ ID NO 4, PYNPQSQGWE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKVVPRRK) and/or HCINp5 (10aa) (SEQ ID NO 15, LLWKGEGAW), preferably with high affinity and/or high specificity;
- the protease (PR) of HIV-1 and HIV-2 variants or portions thereof. In particular, the aptamer is capable of binding to the HCPRp1: LLDTGADD (8aa) and/or HCPRp2: GGIGGFI (7 aa); preferably with high affinity and/or high specificity; or
- the p24 capsid protein of HIV-1 and HIV-2 variants or portions thereof. In particular, the aptamer is capable of binding to the HCCAp1 = PS peptide: PRGSDIAGTTS (11 aa, residues 99-109 P24), HCCAp2 = TC peptide: TLLVQNANPDC (11 aa, residues 188-198 P24), HCCAp3 = PK peptide: PRTLNAWVK (9 aa, residues 17-25 P24), HCCAp4 = QF peptide: QGPKEPFRDYVDRF (14 aa, residues 155-168 P24), HCCAp5 = KT peptide: KAFSPEVIPMFSALSEGATPQDLNT (25 aa, residues 30-54 P24) and/or HCCAp5 = KC peptide: KFGAEVVPGFQALSEGCTPYDINQMLNC (28 aa, residues 29-57 P26), preferably with high affinity and/or high specificity.

In various embodiments, the aptamer is capable of distinguishing between HIV of different HIV variants.

Advantageously, embodiments of the aptamer possess high affinity for HIV, in particular for the integrase (IN) of HIV-1 and HIV-2 variants or portions thereof. More particularly, for the HCINp1 (11 aa) (SEQ ID NO 4, PYNPQSQGWE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKVVPRRK) and/or HCINp5 (10aa) (SEQ ID NO 15, LLWKGEGAW).

In various embodiments, the aptamer has a dissociation constant (K_{D}) of no more than about 14 nM, no more than about 13 nM, no more than about 12 nM, no more than about 1 1 nM, no more than about 10 nM, no more than about 9 nM, no more than about 8 nM, no more than about 7 nM, no more than about 6 nM, no more than about 5 nM, no more than about 4 nM, no more than about 3 nM, no more than about 2 nM or no more than about 1 nM. In various embodiments, the aptamer has a K_{D} of no more than about 800 pM, no more than about 600 pM, no more than about 400 pM, no more than about 300 pM, no more than about 250 pM, no more than about 200 pM, no more than about 190 pM, no more than about 180 pM, no more than about 170 pM, no more than about 160 pM, no more than about 150 pM, no more than about 140 pM, no more than about 130 pM, no more than about 120 pM, no more than about 1 10 pM, no more than about 100 pM, no more than about 90 pM, no more than about 80 pM, no more than about 70 pM, no more than about 60 pM, no more than about 50 pM, no more than about 40 pM, no more than about 30 pM, no more than about 20 pM or no more than about 10 pM.

In various embodiments, the aptamer comprises a sequence as set forth in any of SEQ ID NO 1 to 3 or SEQ ID NO 16 to 18, or a sequence sharing at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity thereto, or a sequence differing by about one, about two, about three, about four, about five, about six, about seven, about eight, about nine, about ten, about 1 1 , about 12, about 13, about 14, about 15, about 16, about 17, about 18, about 19, about 20, about 21 , about 22, about 23, about 24 or about 25 bases or nucleotides thereto, or portions thereof, optionally linear portions thereof. In some embodiments, the aptamer comprises a sequence differing by no more than about one, no more than about two, no more than about three, no more than about four, no more than about five, no more than about six, no more than about seven, no more than about eight, no more than about nine, no more than about ten, no more than about 1 1 , no more than about 12, no more than about 13, no more than about 14, no more than about 15, no more than about 16, no more than about 17, no more than about 18, no more than about 19, no more than about 20, no more than about 21 , no more than about 22, no more than about 23, no more than about 24 or no more than about 25 bases or nucleotides with a sequence as set forth in SEQ ID NO 1 to 3, or portions thereof, optionally linear portions thereof.
- AptlK5.1F = Apt1-HCINp4 (SEQ ID NO 16): 5'-GAGTCTATACGTGCAGCGGGCGTTAATTGAGTGTTATTCCGCGGGGGAC GGAAGTCGGGCGTCCTGGACTTAGGCA-3'
- AptIK5.3F = Apt2-HCINp4 (SEQ ID NO 17): 5'-GAGTCTATACGTGCAGCGGGCTGTTAGAGCTGGTCCCGGAGGTTTAGGG GGTATTTTGGCGTCCTGGACTTAGGCA-3
- AptLV6.2R = Apt1-HCINp5 (SEQ ID NO 18): 5'-TGCCTAAGTCCAGGACGCCACTGTCTCATCACACGACTGCACCCCTACC TATCCCCCACGCTGCACGTATAGACTC-3

### Aptamers K_{D} of SEQ ID NO 16 to 18

- AptIK5.1F = Apt1-HCINp4 (SEQ ID NO 16): 208.7 ± 57.77 nM
- AptIK5.3F = Apt2-HCINp4 (SEQ ID NO 17): 108.7 ± 21.3 nM
- AptLV6.2R = Apt1-HCINp5 (SEQ ID NO 18): 44.49 ± 9.47 nM

In various embodiments, the aptamer comprises a sequence as set forth in any of SEQ ID NO 8 to 9, or a sequence sharing at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity thereto, or a sequence differing by about one, about two, about three, about four, about five, about six, about seven, about eight, about nine, about ten, about 1 1 , about 12, about 13, about 14, about 15, about 16, about 17, about 18, about 19, about 20, about 21 , about 22, about 23, about 24 or about 25 bases or nucleotides thereto, or portions thereof, optionally linear portions thereof. In some embodiments, the aptamer comprises a sequence differing by no more than about one, no more than about two, no more than about three, no more than about four, no more than about five, no more than about six, no more than about seven, no more than about eight, no more than about nine, no more than about ten, no more than about 1 1 , no more than about 12, no more than about 13, no more than about 14, no more than about 15, no more than about 16, no more than about 17, no more than about 18, no more than about 19, no more than about 20, no more than about 21 , no more than about 22, no more than about 23, no more than about 24 or no more than about 25 bases or nucleotides with a sequence as set forth in SEQ ID NO 8 to 9, or portions thereof, optionally linear portions thereof.
AptGI6.1F = Apt1-HCPRp2 (SEQ ID NO 8): 5'-GCGGATGAAGACTGGTGTAGTCCGGCGGGTTGGTTGGGTCGGGTTTTCTTTTTT TGGTGCCCTAAATACGAGCAAC-3'
AptGI6.16F = Apt2-HCPRp2 (SEQ ID NO 9):5'-GCGGATGAAGACTGGTGTGCAGTTGGCGTTGTGTTTGTCCGTGTGTTTCTTGGC GTTTGCCCTAAATACGAGCAAC-3'

In various embodiments, the aptamer comprises a sequence as set forth in any of SEQ ID NO 10 to 14, or a sequence sharing at least about 70%, at least about 75%, at least about 80%, at least about 85%, at least about 90%, at least about 91 %, at least about 92%, at least about 93%, at least about 94%, at least about 95%, at least about 96%, at least about 97%, at least about 98% or at least about 99% sequence identity thereto, or a sequence differing by about one, about two, about three, about four, about five, about six, about seven, about eight, about nine, about ten, about 1 1 , about 12, about 13, about 14, about 15, about 16, about 17, about 18, about 19, about 20, about 21 , about 22, about 23, about 24 or about 25 bases or nucleotides thereto, or portions thereof, optionally linear portions thereof. In some embodiments, the aptamer comprises a sequence differing by no more than about one, no more than about two, no more than about three, no more than about four, no more than about five, no more than about six, no more than about seven, no more than about eight, no more than about nine, no more than about ten, no more than about 11, no more than about 12, no more than about 13, no more than about 14, no more than about 15, no more than about 16, no more than about 17, no more than about 18, no more than about 19, no more than about 20, no more than about 21, no more than about 22, no more than about 23, no more than about 24 or no more than about 25 bases or nucleotides with a sequence as set forth in SEQ ID NO 10 to 14, or portions thereof, optionally linear portions thereof.

| **Detected protein** | **Name** | **Sequece 5'-3'** | **(K_{D})** |
|---|---|---|---|
| P24/P26 capsid protein (VIH-1/VIH-2) | **AptPK9.12F = Apt1-HCCAp3** (SEQ ID NO 10) | AGACAAGTGCTGGGAGAT CGGTTTGGTTACTATCGG GTGGGCGGGCGGGTGCC TTCGGACTTTCCCATTCC GCATT | 78,37±16,43 nM |
| | **AptPK9.14F = Apt2-HCCAp3** (SEQ ID NO 11) | AGACAAGTGCTGGGAGAT GTGAGGGTGGGCGGGTG GGTTGGCATTTCGTTTCG CTTTATACTTTCCCATTCC GCATT | 40,26 ± 11,29 nM |
| P24 capsid protein (VIH-1) | **AptQF5.1**F* **= Apt1-HCCAp4** (SEQ ID NO 12) | AGACAAGTGCTGGGAGAT CGTGGGCGGGTGGGTGG GGTTCGCTTTGCTCCATG GCTGCACTTTCCCATTCC GCATT | |
| | **AptQF5.3F = Apt2-HCCAp4** (SEQ ID NO 13) | AGACAAGTGCTGGAGAT CCGGGGTGGGTGGGGGG GGGTTCGTTCTCTTTGGG TGGTACTTTCCCATTCCG CATT | 73,62±14,75 nM |
| | **AptQF5.9F** = **Apt3-HCCAp4** (SEQ ID NO 14) | AGACAAGTGCTGGGAGAT CGCTGGGTGGGTTGGGT GGGTTGGTTTTTTGGGTT TTTGGACTTTCCCATTCC GCATT | 157,5±39,19 nM |

| | | | |
|---|---|---|---|
| **Affinity assays in progress* | | | |

In various embodiments, the aptamer may be chemically modified.

In some embodiments, the aptamer comprises a chemical modification at its terminus or termini. In some embodiments, the aptamer comprises a chemical modification at its 3' end. In some embodiments, the aptamer comprises a chemical modification at its 5' end.

In some embodiments, modification(s) may be made to the nucleotide unit(s) of the aptamer. For example, modification(s) may be made to the ribose or sugar moiety. In some examples, modification is made at 2' position e.g. 2'- fluoro, 2'-methoxy, 2'-0-methyl and/or 2'-amino modification. In some examples, 2'-methoxy modification comprising deoxyribose modification at 2'-H and 2'-0- methyl modification comprising ribose modification at 2'-OH result in the same chemical structure. In some examples, the modification comprises a 4-thio-sugar modification. In some examples, the aptamer may be composed of or may comprise one or more nucleotide analogues such as peptide nucleic acid (PNA), locked nucleic acid (LNA), morpholino nucleotide, threose nucleic acid (TNA), glycol nucleic acid (GNA), arabinose nucleic acid (ANA), 2'-deoxy-2'-fluoro-b-D- arabinonucleic acid (2' F-ANA), 2'-fluoroarabinose nucleic acid (FANA), 2'-deoxy- 2'-fluororibonucleic acid (2'-F RNA or FRNA) cyclohexene nucleic acid (CeNA), anhydrohexitol nucleic acid (FINA), unlocked nucleic acid (UNA), (4' 6') linked oligo 2',3'-dideoxy-b-D-glucopyranose nucleic acid (homo-DNA or hDNA), xylonucleic acid (XyNA), deoxy-xylonucleic acids (dXyNA), aminoallyl uridine (aa- UTP), derivatives thereof and the like. In some examples, the aptamer may be composed of or may comprise L-ribose-based nucleotide.

In some embodiments, the modification comprises modification to a phosphate linkage part or a phosphodiester linkage. In some examples, the aptamer comprises one or more of a phosphorothioate linkage, a boranophosphate linkage, a methylphophonate, a phosphorthioate analogue, replacement to triazole linkage and the like.

In one example, the aptamer comprises phosphoramidite nucleotides.

In various embodiments, the modification(s) allows the aptamers to be stabilized against nucleic acid-cleaving/degrading enzymes such as nucleases or DNases. In various embodiments, the modification(s) increase the half-life of the aptamer e.g. in a biological sample such as human blood or serum. In various embodiments, the modification(s) allows the modified aptamers to gain desirable properties. In some embodiments, the modification(s) allows the modified aptamers to acquire desirable pharmacology and/or pharmacokinetic properties. In various embodiments, the modified aptamers are stable in the human body. In various embodiments, the modified aptamers possess desirable systemic clearance properties e.g. low glomerular filtration rate. In various embodiments, the modification(s) does not substantially reduce the affinity and/or specificity of the aptamers for HIV, in particular for the integrase (IN) of HIV-1 and HIV-2 variants or portions thereof, more particularly for HCINp1 (11 aa) (SEQ ID NO 4, PYNPQSQGWE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKVVPRRK) and/or HCINp5 (10aa) (SEQ ID NO 15, LLWKGEGAW). In various embodiments, the affinity and/or specificity of the modified aptamers for the above does not substantially differ or is not substantially reduced in comparison to that/those of the unmodified aptamers.

In various embodiments, the unnatural base(s) of the aptamer may also be modified. For example, the unnatural base(s) may be modified to have functional groups such as diol, azide, ethynyl and biotin. In one example, Pa is modified to diol-Pa. In one example, Px is modified to diol-Px. In one example, diol-Px is modified to diol-Pa. In various embodiments, an unnatural base comprising a diol group has enhanced affinity and/or specificity to HIV, in particular for the integrase (IN) of HIV-1 and HIV-2 variants or portions thereof, more particularly for HCINp1 (11 aa) (SEQ ID NO 4, PYNPQSQGWE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKVVPRRK) and/or HCINp5 (10aa) (SEQ ID NO 15, LLWKGEGAW), as compared to the unmodified unnatural base devoid of a diol group or a natural base variant. In some embodiments therefore, the unnatural base(s) comprise a diol group(s).

In various embodiments, the aptamer may be attached/linked/conjugated to one or more molecules. In some examples, functional group modification (e.g. amino, thiol, ethyl, diol etc.) facilitates chemical conjugation to the molecule (e.g. a label, a dye, a reporter, a carrier, a fluorophore, a solid support, a drug, polyethylene glycol (PEG), choresterol, albumin or other materials etc.) via linker. In one example, amino modification using NH2-C6-dT is carried out to introduce a reactive amino group to the aptamer to facilitate chemical conjugation. In some embodiments, the aptamer may be conjugated to a carrier molecule or a reporter molecule. A carrier molecule may allow the aptamer conjugated thereto to attain desirable properties. A reporter molecule may allow the aptamer conjugated thereto to be detectable. An example of a carrier or reporter molecule is biotin. Alternative or additional carrier molecule or reporter molecule may also be conjugated to the aptamer. In various embodiments, the conjugation does not substantially reduce the affinity and/or specificity of the aptamers for HIV, in particular for the integrase (IN) of HIV-1 and HIV-2 variants or portions thereof, more particularly for HCINp1 (11 aa) (SEQ ID NO 4, PYNPQSQGWE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKVVPRRK) and/or HCINp5 (10aa) (SEQ ID NO 15, LLWKGEGAVV).

Non-limiting examples of modification/modifiers include amino modifiers (e.g. amino modifier C6, amino modifier C12, amino modifier C6 dT, Uni-Link amino modifier etc.), biotinylation (e.g. biotin, biotin (azide), biotin dT, biotin-TEG, dual biotin, PC biotin, desthiobiotin-TEG etc.), thiol modifications (e.g. thiol modifier C3 S-S, dithiol, thiol modifier C6 S-S etc.), alkyne modifier (e.g. 5' Hexynyl), 5-Octadiynyl dU etc.), Acrydite, adenylation, azide , azide (NHS Ester), cholesterol-TEG, digoxigenin, digoxigenin (NHS ester), l-Linker, fluorophore and dark quencher (e.g. fluorescein, Cy, rhodamine dye, Alexa Fluor dye, ATTO dye, IRDye, FAM, 6- FAM, 6-FAM (NHS ester), 6-FAM (fluorescein), fluorescein dT, Cy3, TAMRA, JOE, JOE (NHS ester), MAX, MAX (NHS ester), TET, Cy5.5, ROX, ROX (NHS ester), TYE 563, Yakima Yellow, HEX, TEX 615, TYE 665, TYE 705, Texas Red-X, Texas Red-X (NHS ester), Lightcycler 640, Lightcycler 640 (NHS Ester), Dy 750, Dy 750 (NHS ester), dark quencher, Iowa Black dark quencher, Iowa Black FQ, Iowa Black RQ, Black Hole Quencher-1 , Black Hole Quencher- 2, Dabcyl etc.), modified base modification (e.g. locked nucleic acid, 2'-0-methoxy-ethyl base (2'-MOE), 2'-0-methyl RNA base, fluoro base, 2- aminopurine, 5-Bromo dU, deoxyUridine, 2,6-Diaminopurine (2-Amino-dA), dideoxy-C, deoxylnosine, hydroxymethyl dC, inverted dT, iso-dG, iso-dC, inverted dideoxy-T, 5-methyl dC, 5-nitroindole etc.), phosphorylation modification, spacer modification (e.g. C3 spacer, hexanediol, 1',2'-dideoxyribose (dSpacer), PC spacer, spacer 9, spacer 18 etc.), click chemistry modification (e.g. (i) 5', Internal, or 3' azide, (ii) 5', Internal, or 3' azide (NHS ester), (iii) 5' hexynyl, (iv) 5', Internal, or 3' 5-Octadiynyl dU, (v) 5', or Internal biotin, (vi) 5', or internal biotin (azide), (vii) 5', or Internal 6-FAM , (viii) 5', or Internal 6-FAM (azide), (viiii) 5', or Internal 5-TAMRA, (x) 5', or Internal 5-TAMRA (azide) etc.), phosphorothioate bonds modification (e.g. phosphorothioated DNA bases, phosphorothioated RNA bases, phosphorothioated 2'-O-methyl bases, phosphorothioated Affinity Plus (locked nucleic acid) bases etc.) and the like. Embodiments of the aptamers may have some inhibitory effects on HIV. Embodiments of the aptamers may also show desirable pharmacology and/or pharmacokinetic properties (e.g. stability and/or low systemic clearance etc.).

In various embodiments, the aptamer is capable of recognizing and/or binding to a HIV protein, in particular to the integrase (IN) of HIV-1 and HIV-2 variants or portions thereof, more particularly for HCINp1 (11 aa) (SEQ ID NO 4, PYNPQSQGWE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKVVPRRK) and/or HCINp5 (10aa) (SEQ ID NO 15, LLWKGEGAW), sharing at least about 95%, at least about 95.5%, at least about 96%, at least about 96.1 %, at least about 96.2%, at least about 96.3%, at least about 96.4%, at least about 96.5%, at least about 96.6%, at least about 96.7%, at least about 96.8%, at least about 96.9%, at least about 97%, at least about 97.1 %, at least about 97.2%, at least about 97.3%, at least about 97.4%, at least about 97.5%, at least about 97.6%, at least about 97.7%, at least about 97.8%, at least about 97.9%, at least about 98%, at least about 98.1 %, at least about 98.2%, at least about 98.3%, at least about 98.4%, at least about 98.5%, at least about 98.6%, at least about 98.7%, at least about 98.8%, at least about 98.9% or at least about 99% sequence identity/homology with any of the sequences set forth in SEQ ID NO 1 to 3. In various embodiments, the aptamer is capable of recognizing and/or binding to a HIV protein, in particular to the integrase (IN) of HIV-1 and HIV-2 variants or portions thereof, more particularly for HCINp1 (11 aa) (SEQ ID NO 4, PYNPQSQGWE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKVVPRRK) and/or HCINp5 (10aa) (SEQ ID NO 15, LLWKGEGAVV), having no more than about 15 amino acid difference, no more than about 14 amino acid difference, no more than about 13 amino acid difference, no more than about 12 amino acid difference, no more than about 1 1 amino acid difference, no more than about 10 amino acid difference, no more than about 9 amino acid difference, no more than about 8 amino acid difference, no more than about 7 amino acid difference, no more than about 6 amino acid difference, no more than about 5 amino acid difference, no more than about 4 amino acid difference, no more than about 3 amino acid difference, no more than about 2 amino acid difference or no more than about 1 amino acid difference with any one of the sequences set forth in SEQ ID NO 1 to 3 or SEQ ID NO 16 to 18.

In various embodiments, the aptamer is capable of recognizing and/or binding to a HIV protein, in particular to the protease (PR) of HIV-1 and HIV-2 variants or portions thereof. In particular, the aptamer is capable of binding to the HCPRp1: LLDTGADD (8aa) and/or HCPRp2: GGIGGFI (7 aa), preferably with high affinity and/or high specificity, sharing at least about 95%, at least about 95.5%, at least about 96%, at least about 96.1 %, at least about 96.2%, at least about 96.3%, at least about 96.4%, at least about 96.5%, at least about 96.6%, at least about 96.7%, at least about 96.8%, at least about 96.9%, at least about 97%, at least about 97.1 %, at least about 97.2%, at least about 97.3%, at least about 97.4%, at least about 97.5%, at least about 97.6%, at least about 97.7%, at least about 97.8%, at least about 97.9%, at least about 98%, at least about 98.1 %, at least about 98.2%, at least about 98.3%, at least about 98.4%, at least about 98.5%, at least about 98.6%, at least about 98.7%, at least about 98.8%, at least about 98.9% or at least about 99% sequence identity/homology with any of the sequences set forth in SEQ ID NO 8 to 9. In particular, the aptamer is capable of binding to the HCPRp1: LLDTGADD (8aa) and/or HCPRp2: GGIGGFI (7 aa), preferably with high affinity and/or high specificity, having no more than about 15 amino acid difference, no more than about 14 amino acid difference, no more than about 13 amino acid difference, no more than about 12 amino acid difference, no more than about 1 1 amino acid difference, no more than about 10 amino acid difference, no more than about 9 amino acid difference, no more than about 8 amino acid difference, no more than about 7 amino acid difference, no more than about 6 amino acid difference, no more than about 5 amino acid difference, no more than about 4 amino acid difference, no more than about 3 amino acid difference, no more than about 2 amino acid difference or no more than about 1 amino acid difference with any one of the sequences set forth in SEQ ID NO 8 to 9.

In various embodiments, the aptamer is capable of recognizing and/or binding to a HIV protein, in particular to the p24 capsid protein of HIV-1 and HIV-2 variants or portions thereof. In particular, the aptamer is capable of binding to the HCCAp1 = PS peptide: PRGSDIAGTTS (11 aa, residues 99-109 P24), HCCAp2 = TC peptide: TLLVQNANPDC (11 aa, residues 188-198 P24), HCCAp3 = PK peptide: PRTLNAWVK (9 aa, residues 17-25 P24), HCCAp4 = QF peptide: QGPKEPFRDYVDRF (14 aa, residues 155-168 P24), HCCAp5 = KT peptide: KAFSPEVIPMFSALSEGATPQDLNT (25 aa, residues 30-54 P24) and/or HCCAp5 = KC peptide: KFGAEVVPGFQALSEGCTPYDINQMLNC (28 aa, residues 29-57 P26, preferably with high affinity and/or high specificity., sharing at least about 95%, at least about 95.5%, at least about 96%, at least about 96.1 %, at least about 96.2%, at least about 96.3%, at least about 96.4%, at least about 96.5%, at least about 96.6%, at least about 96.7%, at least about 96.8%, at least about 96.9%, at least about 97%, at least about 97.1 %, at least about 97.2%, at least about 97.3%, at least about 97.4%, at least about 97.5%, at least about 97.6%, at least about 97.7%, at least about 97.8%, at least about 97.9%, at least about 98%, at least about 98.1 %, at least about 98.2%, at least about 98.3%, at least about 98.4%, at least about 98.5%, at least about 98.6%, at least about 98.7%, at least about 98.8%, at least about 98.9% or at least about 99% sequence identity/homology with any of the sequences set forth in SEQ ID NO 10 to 14. In various embodiments, the aptamer is capable of recognizing and/or binding to a HIV protein, in particular to the HCCAp1 = PS peptide: PRGSDIAGTTS (11 aa, residues 99-109 P24), HCCAp2 = TC peptide: TLLVQNANPDC (11 aa, residues 188-198 P24), HCCAp3 = PK peptide: PRTLNAWVK (9 aa, residues 17-25 P24), HCCAp4 = QF peptide: QGPKEPFRDYVDRF (14 aa, residues 155-168 P24), HCCAp5 = KT peptide: KAFSPEVIPMFSALSEGATPQDLNT (25 aa, residues 30-54 P24) and/or HCCAp5 = KC peptide: KFGAEVVPGFQALSEGCTPYDINQMLNC (28 aa, residues 29-57 P26, having no more than about 15 amino acid difference, no more than about 14 amino acid difference, no more than about 13 amino acid difference, no more than about 12 amino acid difference, no more than about 1 1 amino acid difference, no more than about 10 amino acid difference, no more than about 9 amino acid difference, no more than about 8 amino acid difference, no more than about 7 amino acid difference, no more than about 6 amino acid difference, no more than about 5 amino acid difference, no more than about 4 amino acid difference, no more than about 3 amino acid difference, no more than about 2 amino acid difference or no more than about 1 amino acid difference with any one of the sequences set forth in SEQ ID NO 10 to 14.

In some embodiments therefore, the aptamer is capable of distinguishing between HIV variants. In one embodiment, the aptamer is capable of binding specifically to a single serotype of HIV selected from the group consisting of serotype 1 or serotype 2. More preferably, the aptamer is capable of binding specifically to HIV-1, more particularly to any of the four groups; M, N, O and P. Preferably, to group M and more preferably to any of the distinct subtypes; A, B, C, D, F, G, H, J, K and L.

In various embodiments, there is provided a mixture/combination of aptamers that are specific to different serotypes, the mixture comprising at least two, at least three, or at least four of the aptamers. For example, the mixture/combination may comprise an aptamer that is specific to HIV, more particularly for HCINp1 (11 aa) (SEQ ID NO 4, PYNPQSQGWE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKVVPRRK) and/or HCINp5 (10aa) (SEQ ID NO 15, LLWKGEGAVV). For example, the mixture/combination may comprise any three of the following: an aptamer of SEQ ID NO 1, an aptamer of SEQ ID NO 2, an aptamer of SEQ ID NO 3 or any combination thereof (please see the examples for the specific combinations disclosed therein). The same considerations can be made to any of aptamers of SEQ ID NO 8 to 14.

In various embodiments, there is provided a method of identifying a HIV infection in a subject, the method comprising contacting a sample of the subject with the aptamer or the mixture of aptamers. In some embodiments, the method further comprises detecting a binding event at the aptamer(s). In various embodiments therefore, there is provided a method of identifying a HIV infection in a subject, the method comprising: contacting a sample of the subject with the aptamer or the mixture of aptamers and detecting a binding event at the aptamer(s).

In various embodiments, the method may be implemented in the form of a binding assay. In various embodiments, the method may be in the form of a "sandwich" assay, where a first binder or capturing agent (e.g. an aptamer) serves to capture a target analyte (e.g. a HIV protein) and a second binder or detector agent (e.g. a further reporter aptamer) is used to detect the captured target analyte. Examples of such assays include enzyme immunoassays (EIA) / enzyme-linked immunosorbent assay (ELISA), enzyme-linked aptamers assays (ELAA) /enzyme-linked oligonucleotide assay (ELONA), radioimmunoassay RIA, strip assays, lateral flow assays (LFA), bio-layer interferometry (BLI), the detection through Surface plasmon resonance (SPR), colorimetric changes using gold nanoparticle aggregations, voltammetric, and electrochemical techniques and the like. The suitability of the specific assay to be used would be within the purview of the skill of the person skilled in the art.

In various embodiments, the method comprises an ELISA method, such as, but is not limited to direct ELISA, indirect ELISA, competitive ELISA, sandwich ELISA, and the like. In some embodiments, the method comprises a sandwich ELISA method. The sandwich ELISA may be competitive or non-competitive. In some examples, the aptamer may be labelled, for example with biotin, and may be bound to a solid phase e.g. a bead, a surface of a well or other containment body, a chip or a strip, for capturing any HIV protein and a further aptamer of the HIV protein is used for detecting any captured HIV protein. In some examples, a first aptamer of the HIV protein is used as a primary detector agent and a second aptamer against the protein is used as a secondary detector agent. The detector agent may be labelled, for example, with a dye, radioisotope or a reactive or catalytically active moiety. In one example, the detector agent is labelled with horseradish peroxidase (HRP) and tetramethylbenzidine (TMB) substrate may be added for visualization. It will be appreciated that other suitable labels and substrates may also be used. In some examples, a plate (e.g. microplate, microtiter plate etc.) coated with streptavidin is used for immobilising the biotin-labelled aptamer, which is then used for capturing a HIV protein, and a labelled detector agent (or a primary detector agent and a labelled secondary detector agent) is used for detection. The detector agent may be a further aptamer or an anti-immunoglobulin such as anti-IgG (e.g. IgG1, IgG2, IgG3 or lgG4), anti-IgM or anti-IgA. In some examples, the anti-immunoglobulin comprises anti-human IgM, anti-goat IgG, anti-rabbit IgG, and/or anti-mouse IgG. When a primary detector is labelled with biotin, the secondary agent may be an anti-biotin antibody or streptavidin. In some embodiments, a binding event at any of the aptamer(s) is indicative of a current HIV infection in the subject. In some examples, a binding event is indicated by a chemiluminescence or colorimetric signal/readout/output e.g. resulting from HRP conversion of TMB. In some embodiments, where the aptamer comprises an aptamer that is specific to or that binds specifically to a single HIV serotype, the binding event is indicative of a current HIV infection of said serotype in the subject. For example, where the aptamer is an aptamer specific to HIV-1, detection of a binding event at the aptamer (e.g. observation of a colorimetric output in a HRP-TMB system) when the aptamer is contacted with a subject's sample is indicative that the subject has HIV-1 - protein in his sample, and therefore indicative that the subject has that specific serotype.

In one example, the method comprises a non-competitive binding assay method. In one example, the method comprises a non-competitive ELISA method. In one example, the method comprises direct detection of HIV protein, in the sample.

Without wishing to be bound by theory, it is believed that HIV antigen binding protein, such as the integrase (IN) of HIV-1 and HIV-2 variants or portions thereof, more particularly the HCINp1 (11 aa) (SEQ ID NO 4, PYNPQSQGWE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKVVPRRK) and/or HCINp5 (10aa) (SEQ ID NO 15, LLWKGEGAW), is generated by a subject's body in response to HIV infection.

In various embodiments, the method further comprises administering a HIV treatment regimen to the subject if the subject is indicated for a current HIV infection.

In various embodiments, the sample comprises a biological sample. In various embodiments, the biological sample comprises a fluid biological sample or a liquid biological sample. The fluid biological sample or liquid biological sample may be blood, serum, plasma, sputum, lavage fluid, cerebrospinal fluid, urine, semen, sweat, tears, saliva, and the like. In some embodiments, the fluid biological sample or liquid biological sample comprises whole blood, blood serum, blood plasma or processed fractions thereof. In some embodiments, the fluid biological sample comprises blood serum or blood plasma.

In various embodiments, the method comprises a diagnostic method. For example, in a direct HIV protein detection method, a binding event at an aptamer may be indicative of HIV infection in the subject.

In various embodiments, the method comprises a prognosis method.

In various embodiments, the method has high sensitivity and/or specificity. In various embodiments, the method has a sensitivity of at least about 50%, at least about 55%, at least about 60%, at least about 65%, at least about 70%, at least about 75%, at least about 80%, at least about 81%, at least about 82%, at least about 83%, at least about 84%, at least about 85%, at least about 86%, at least about 87%, at least about 88%, at least about 89%, at least about 90%, at least about 91 %, at least about 92%, at least about 93%, at least about 94%, at least about 95%, at least about 96%, at least about 97%, at least about 98%, at least about 99% or at least about 100%.

In various embodiments, the method has a specificity of at least about 50%, at least about 55%, at least about 60%, at least about 65%, at least about 70%, at least about 75%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99% or at least about 100%.

In various embodiments, the method comprises an in vitro or an ex vivo method.

In various embodiments, there is provided a kit for identifying a HIV infection in a subject, the kit comprising the aptamer or the mixture of aptamers. In some embodiments, the aptamer or the mixture of aptamers is provided in the form of a plate coated with the aptamer or the mixture of aptamers for capturing HIV protein. In some embodiments, the kit further comprises a HIV protein. In some embodiments, the kit comprises a detector agent and/or a capturing agent, preferably in the form of aptamers.

The kit may be a diagnostic kit or a prognostic kit.

In various embodiments, the subject comprises a mammal. In various embodiments, the subject comprises a human subject.

In various embodiments, there is provided a nucleic acid molecule comprising a sequence set out in SEQ ID NO 1 to 3 or set out in SEQ ID NO 8 to 14 or set out in SEQ ID NO 16 to 18, or a sequence sharing at least about 70%, at least about 75%, at least about 80%, at least about 85%, at least about 90%, at least about 91%, at least about 92%, at least about 93%, at least about 94%, at least about 95%, at least about 96%, at least about 97%, at least about 98% or at least about 99% sequence identity thereto, or a sequence differing by about one, about two, about three, about four, about five, about six, about seven, about eight, about nine, about ten, about 1 1 , about 12, about 13, about 14, about 15, about 16, about 17, about 18, about 19, about 20, about 21 , about 22, about 23, about 24 or about 25 bases or nucleotides thereto, or portions thereof, optionally linear portions thereof. In some embodiments, the aptamer comprises a sequence differing by no more than about one, no more than about two, no more than about three, no more than about four, no more than about five, no more than about six, no more than about seven, no more than about eight, no more than about nine, no more than about ten, no more than about 1 1 , no more than about 12, no more than about 13, no more than about 14, no more than about 15, no more than about 16, no more than about 17, no more than about 18, no more than about 19, no more than about 20, no more than about 21 , no more than about 22, no more than about 23, no more than about 24 or no more than about 25 bases or nucleotides with a sequence set forth in SEQ ID NO 1 to 3 or set out in SEQ ID NO 8 to 14 or set out in SEQ ID NO 16 to 18, or portions thereof, optionally linear portions thereof.

In some embodiments, the nucleic acid molecule comprises a sequence set forth in SEQ ID NO 1 to 3 or set out in SEQ ID NO 8 to 14 or set out in SEQ ID NO 16 to 18, or a sequence sharing at least 75% sequence identity thereto; or a sequence differing by one, two, three, four, five, six, seven, eight, nine or ten bases thereto; or portions thereof.

In various embodiments, there is provided a method of producing the nucleic acid molecule or the aptamer. In various embodiments, the method comprises a chemical synthesis method. In various embodiments, the method comprises an oligonucleotide synthesis method. In various embodiments, the method comprises a phosphoramidite method of oligonucleotide synthesis. The synthesis can be a solution-phase synthesis or a solid-phase synthesis. In some embodiments, the synthesis comprises a solid-phase synthesis. Other suitable methods of synthesising a nucleic acid molecule or aptamer or oligonucleotide may also be used. For example, the method may comprise a H-phosphonate method and/or a phosphotriester method of oligonucleotide synthesis.

In various embodiments, the method may comprise a step of incorporating one or more modifications to the nucleic acid molecule or the aptamer. The incorporation may be performed during and/or after synthesis of e.g. the oligonucleotide.

In various embodiments, there is provided a method, a kit or a nucleic acid molecule as described herein.

The following examples merely illustrate but do not limit the present invention.

### Examples

### Example 1

### EXPERIMENTAL SECTION

### Selection of highly conserved and exposed IN peptides among HIV variants

To identify highly conserved peptides in the integrase (IN) of HIV-1 and HIV-2 variants, we downloaded one sequence per patient from the Los Alamos National Laboratory HIV Sequence Database (LANL), including all HIV types, groups, subtypes, sub-subtypes, and CRFs. We excluded URFs, sequences with stop codons or interrogation marks. The remaining sequences were aligned using Muscle v3.8.31. We used EpiMolBio, an in-house bioinformatics tool, to identify conserved peptides across all HIV variants. After determining the percentage of sequences with fully conserved peptides per HIV variant, we examined peptide exposure in IN using PyMOL program. The 3D structures of HIV-1 and HIV-2 integrases were generated by AlphaFold 3, using the HXB2 reference sequence (GenBank NC_001802.1) for HIV-1 and the ALI reference sequence (GenBank AF082339.1) for HIV-2.

### Aptamers selection by Systematic Evolution of Ligands by Exponential Enrichment (SELEX) and sequencing by NGS

Synthetic random single stranded DNA (ssDNA) population (P3-RND40) and non-labelled and labelled primers for aptamer's selection were obtained from IBA GmbH (Göttingen, Germany). All the aptamers were synthetized by Biomers GmbH (Göttingen, Germany). IN peptide was synthetized by ProteoGenix (Schiltigheim, France) and recombinant HIV-1 IN (ARP-9420/HRP-20203) was provided by BEI Resources previously known as HIV Reagent Program.

Aptamers were selected by Systematic Evolution of Ligands by Exponential Enrichment (SELEX). For that, we performed iterative rounds of SELEX method as previously reported of ssDNA P3-RND40 library. These oligonucleotides contained a central randomized region of 40 nucleotides flanked by two conserved regions of 18 nucleotides in each end (5'-GCGGATGAAGACTGGTGT(N)40GCCCTAAATACGAGCAAC-3'). The highly-conserved peptide (300 ng) was immobilized on a nitrocellulose membrane and subsequently blocked with PBS-BSA 5% for 1 hour. P3-RND40 was denatured 10 min at 90 °C and then cooled another 10 min on ice. For the initial SELEX round, the peptide-immobilized membrane was incubated with 1 nmol of RND40 (5:1 ratio) in 1 mL of selection buffer (PBS + 1 mM MgCl₂) for 1 hour at RT. After washing three times with 1 mL of selection buffer, the bound aptamer were released in 100 µL of hot PCR reaction mixture and amplified by PCR using primers labeled F3 (5'-GCGGATGAAGACTGGTGT-3') and R3 (5'-GTTGCTCGTATTTAGGGC-3') under the following conditions: 0. 8 µM/primer, 200 µM dNTPs, 3 mM MgCl₂ and 1 U of Taq polymerase (Biotools, Madrid, Spain) at 57°C for 10 cycles. From the fourth round of selection, the incubation time was reduced to 30 min. A nitrocellulose membrane counterselection step was performed before the first three rounds. After the fifth round (R5), purified rounds 3 (R3) and 5 (R5) were sent to the Genomics Unit of the National Center for Cardiovascular Research (CNIC) for next-generation sequencing (NGS). The resulting sequencing files were analyzed to identify overrepresented sequences in both populations.

### Bioinformatic analysis of anti-HIV integrase aptamers

The ssDNA aptamer secondary structure was predicted at 25°C and 37°C using the DNA Folding Form application, with 150mM Na⁺ and 1 mM Mg²⁺ concentrations. We also generated 3D RNA models with the RNAcomposer server, which were then converted back to DNA using the x3DNA server, replacing uracil with thymine and ribose with deoxyribose to create new PDB files. Additionally, G-quadruplex prediction in the ssDNA aptamer's sequences was performed using QGRS Mapper and compared with another in-house bioinformatics tool, EpiMolBio Apt, to assess potential improvements in stability and nuclease resistance.

### Enzyme-linked oligonucleotide assay (ELONA) for aptamers' characterization

To determine the binding capability or affinity (dissociation constant or K_{D}) of the aptamers for recombinant HIV-1 IN detection, an indirect ELONA assay was performed by coating 96-well plates with 100 µL of 1 ng/µL HIV-1 recombinant IN (BEI resources, ARP-9420), and incubated overnight at 4°C on a shaker platform, followed by blocking with PBS/MgCl₂ + 5% BSA. Then, different concentrations of digoxigenin-labeled aptamers (from 6.25 to 1,600 nM) were added, followed by a poly-HRP anti-digoxigenin secondary antibody. The addition of ABTS resulted in a color reaction that could be measured. To assess aptamer sensitivity, similar experiments were performed coating wells with different concentrations of recombinant HIV-1 IN (from 3.13 and 100 ng with 200 ng BSA as negative control (NC), maintaining the aptamer concentration at five times its K_{D}. Each experiment performed by ELONA was conducted using three plates, with three wells per plate for each determination..

To identify the optimal combination of aptamers and assess their sensitivity for recombinant HIV-1 IN detection, we employed a sandwich ELONA. This method, similar to indirect ELONA, involves using a biotin-labelled capture aptamer at a concentration of 0.5 µM, which is added to a streptavidin-coated plate and incubated overnight at 4°C with agitation. The following day, blocking was carried out, and the target protein was added, with subsequent steps following the standard indirect ELONA protocol. We tested all nine possible capture/detector aptamer combinations of the three selected aptamers against integrase, Negative controls included 200 ng BSA protein or PBS/MgCl₂ without recombinant IN protein.

The sensitivity of the best aptamer combination was further evaluated using different concentrations of recombinant HIV-1 IN ranging from 0.03 to 1 ng/µL (3.13 to 100 ng per well). We used the optimal aptamer combination to detect multiple HIV-positive clinical samples (Table 1).

**Table 1. HIV-1-infected samples used in sandwich ELONA using the best combination of aptamers able to detect IN. (*) Viral load tested by Xpert ^{®} HIV-1 Viral Load (Cepheid). VL, viral load; cp/mL, copies of HIV-1 RNA per plasma millilitre; ng, nanogram; P24, HIV-1 capsid protein; P26, HIV-2 capsid protein.**

| | | | | | | **Original sample** | | | **After lysis and/or antigen-antibody dissociation** | | |
|---|---|---|---|---|---|---|---|---|---|---|---|
| | **Code** | **GenBank accession number** | **Sample type** | **Virus type** | **HIV variant** | **P24/P26 (ng/mL)** | **VL (cp/mL)** | **VL (log/mL)** | **IN (ng/mL)** | **VL (cp/mL)** | **VL (log/ mL)** |
| **SB1** | 1650 | AY713407 | | | Group M | 50.10 | 1.26E+09 | 9.10 | 11.48 | 8.38E+08 | 8.92 |
| **SB2** | 11246 | AF457066 | | | Subtype A | 33 | 8.28E+08 | 8.92 | 7.56 | 5.52E+08 | 8.74 |
| **SB3** | 7691 | AY713951 | | | Group M | 100 | 2.51E+09 | 9.40 | 22.92 | 1.67E+09 | 9.22 |
| **SB4** | 7689 | L14576 | | | Subtype 8 | 127.20 | 3.19E+09 | 9.50 | 29.15 | 2.13E+09 | 9.33 |
| **SBS** | 11258 | AY713417 | | | Group M | 91.60 | 2.30E+09 | 9.36 | 20.99 | 1.63E-09 | 9.19 |
| **SB6** | 2913 | AY713413 | Cell-free virus | | Subtype C | 86.60 | 2.18E+09 | 9.34 | 19.85 | 1.45E+09 | 9.16 |
| **SB7** | 1952 | AY713418 | | | Group M | 121.40 | 3.05E+09 | 9.48 | 21.82 | 2.03E+09 | 9.31 |
| **SB8** | 11259 | AF485402 | | | Subtype D | 98.30 | 2.47E+09 | 9.39 | 22.53 | 1.64E+09 | 9.22 |
| **SBS** | 11271 | AY713422 | | | Group M | 42.70 | 1.07E+09 | 9.03 | 9.79 | 7.14E+08 | 8.85 |
| **SB10** | 11269 | AY713424 | | | CRF01_AE | 42.58 | 1.07E+09 | 9.03 | 9.76 | 7.12E+08 | 8.85 |
| **SB11** | 11279 | AY371124 | | HIV-1 | Group M | 118.90 | 2.98E+09 | 9.47 | 27.25 | 1.99E+09 | 9.30 |
| **5812** | 11277 | AY371122 | | | CRF02_AG | 116.5 | 2.92E+09 | 9.47 | 26.70 | 1.95E+09 | 9.29 |
| **S1** | - | Unknown | Sera | | Group M Subtype B | 2.60E-03 | 6.46E+04 | 4.81 | 2.95E-04 | 2.17E+04 | 4.34 |
| **S2** | | | | | Unknown | 2.09E-02 | 5.25E+05 | 5.72 | 2.40E-03 | 1.75E+05 | 5.24 |
| **S3** | | | | | | 5.02E-02 | 1.26E+06 | 6.10 | 5.75E-03 | 4.20E+05 | 5.62 |
| **S4** | | | | | | 5.02E-02 | 1.26E+06 | 6.10 | 5.75E-03 | 4.20E+05 | 5.62 |
| **P1** | GE-NT1 | OL470821 | Plasma | | Group M CRF02_AG | 3.16E-02 | *7.94E+05 | 5.90 | 3.63E-03 | 2.65E+05 | 5.42 |
| **P2** | GE-AN54 | OL470778 | | | Group M CRF06_cpx | 2.51E-02 | *6.31E+05 | 5.80 | 2.88E-03 | 2.10E+05 | 5.32 |

These included: 1) twelve cell-free HIV-1 supernatants from BEI Resources covering HIV-1 group M subtypes A, B, C, D, and CRF01_AE and CRF02_AG; 2) Four HIV-positive sera from the Hospital Universitario Ramon y Cajal, one of which was known to be group M subtype B; 3) Two HIV-infected plasma samples from Equatorial Guinea with CRF02_AG and CRF06_cpx forms, respectively. Negative controls included HIV-negative plasma and serum samples and PBS/MgCl₂ for cell-free supernatants. We also included an HIV negative hepatitis C virus (HCV) positive serum (2.20·10⁷ HCV virions/mL) provided by the Hospital Universitario Ramon y Cajal to evaluate the specificity of this aptamer combination. All clinical samples required a previous lysis in a Triton/Tris-HCl buffer for 30 minutes before testing in ELONA. After lysis, all serum and plasma samples were treated with a Glycine-HCl buffer to dissociate antigen-antibody complexes, followed by neutralization with NaOH. We used 100 µL of these pre-treated samples for the sandwich ELONA assay.

### Statistical analysis

Data, presented as mean ± SEM of six to nine independent measurements, were analyzed using GraphPad Prism 8.0. Affinity data were fitted to a one-site binding model (y = (x - Bmax) / (x + K_{D})), where Bmax is the maximum binding and K_{D} is the concentration for half-maximal binding. Statistical significance was determined by ANOVA with Dunnett's test or unpaired t-test with Welch's correction, depending on the experiment. A p-value <0.05 was considered significant.

### RESULTS AND DISCUSSION

### Selection of highly-conserved and exposed IN peptide for aptamers selection

Previously published studies have reported amino acid conservation rates among HIV-1 and HIV-2 integrases (INs). Using the EpiMolBio tool, we identified four highly conserved IN peptides: HCINp1 (11 aa), HCINp2 (9 aa), HCINp3 (9 aa), and HCINp4 (8 aa). To assess the degree of conservation of these peptides among HIV variants at the time of the study, we downloaded 13,606/6,436 HIV-1/HIV-2 IN sequences of 142/8 HIV-1/HIV-2 variants from LANL. Then, we quantified the percentage of sequences in each HIV variant that presented 100% conservation for HCINp1 (113/4 HIV-1/HIV-variants), HCINp2 (103/0 HIV-1/HIV-2 variants), HCINp3 (122/2 HIV-1/HIV-2 variants), and HCINp4 (110/1 HIV-1/HIV-2 variants) **(****Figure 1****).** To explore the peptides exposure in the IN, we visualized 3D HIV-1 and HIV-2 IN structures **(****Figure 2****).**

### Aptamers characterization recognizing highly conserved and exposed peptide HC-INp1.

We selected 3 ssDNA aptamers directed to the highly conserved and exposed peptide HC-INp1 (Apt1-HCINp1, Apt2-HCINp1, and Apt3-HCINp1).

Their sequences are as follows:
AptPE5.1F = Apt1-HCINp1 (SEQ ID NO 1) 5'-GCGGATGAAGACTGGTGTGGGTGTGGTGTTGGGTATTGGGCGTGTGCGCTGTG GTGGCTGCCCTAAATACGAGCAAC-3'
AptPE5.4F = Apt2-HCINp1 (SEQ ID NO 2) '5'-GCGGATGAAGACTGGTGTGCGGTGGTGGGTTGGGTGGGTGGGTTTGCGGGTT GCGTTGGCCCTAAATACGAGCAAC-3'
AptPE6.8F = Apt3-HCINp1 (SEQ ID NO 3) 5'-GCGGATGAAGACTGGTGTTGGTTGGGTGGCGGTTAGGGGGTCTGGAGGTGGG GTTGGTGCCCTAAATACGAGCAAC-3'

Their sequences were analyzed using three bioinformatics tools to infer their stability and predicted secondary structure: QGRS Mapper, DNA Folding, and EpiMolBio Apt **(Table 2).** DNA Folding revealed than Apt1-HCINp1 was the most stable, followed by Apt2-HCINp1 and Apt3-HCINp1, based on free energy at 25°C and 37°C. QGRS Mapper and EpiMolBio Apt indicated that Apt2-HCINp1 and Apt3-HCINp1 presented a higher propensity for G-quadruplex formation due to more QGRS motifs and higher G-scores compared to Apt1-HCINp1, suggesting greater stability. The aptamers exhibited different predicted secondary structures, implying varying affinities for binding HIV-1 IN **(****Figure 3****).**

**Table 2. Bioinformatics analyses of G-quadruplex and Gibbs free energy with Apt1-HCINp1, Apt2-HCINp1 and Apt3-HCINp1. NT, nucleotides; %GC, guanine-cytosine percentage; N°, number; QGRS, quadruplex forming G-rich sequence(s); MED, medium; MAX, maximum; ΔG, Gibbs free energy.The given QGRS location is the first nucleotide of the QGRS. Bioinformatic tools used: QGRS Mapper, DNA Folding, and EpiMolBio Apt.**

| **APTAMER** | **LENGTH (NT)** | **%GC** | **N° QGRS** | **MED. G-SCORE** | **MAX. G-SCORE** | **N° QGRS WITH MAX. G-SCORE** | **QGRS LOCATIONS WITH MAX. G-SCORE** | **ΔG (25°C)** | **ΔG (37°C)** |
|---|---|---|---|---|---|---|---|---|---|
| Apt1-HCINp1 | 77 | 58.44% | 43 | 15.07 | 20 | (x5) | 14 (x2) and 19 (x3) | -6.63 | -2.73 |
| Apt2-HCINp1 | 76 | 60.53% | 412 | 14.45 | 41 | (x1) | 27 | -4.88 | -2.43 |
| Apt3-HCINp1 | 76 | 59.21% | 711 | 12.92 | 21 | (x3) | 20,28 and 38 | -4.60 | -1.66 |

Regarding affinity, indirect ELONAs using 100ng HIV-1 IN per well, revealed that the Apt1-HCINp1 showed a K_{D} of 92.55 ± 29.2 nM, Apt2-HCINp1 a K_{D} of 30.95 ± 4.79 nM and Apt3-HCINp1 a K_{D} of 196.4 ± 68.44 nM for HIV-1 IN. **(****Figure 4A****).** Thus, the aptamer with the highest affinity for HIV integrase was Apt2-HCINp1 due to a lower dissociation constant. To assess sensitivity, indirect ELONA was performed on plates coated with recombinant HIV-1 IN between 3.13 and 100 ng along with 200 ng BSA as negative control (NC). Apt1-HCINp1, Apt2-HCINp1 and Apt3-HCINp1 detected HIV-1 IN in a concentration-dependent manner up to 12.5 ng, with significant differences compared to BSA (p = 0.0095, p = 0.0053 and p = 0.0311, respectively) **(****Figure 4B****).** This amount of recombinant HIV-1 IN would correspond to 4.56·10⁸ virions/well (or VL of 8.96 log copies HIV-1 RNA/well)

### Aptamers best combinations and their sensitivity

All possible combinations of the three selected aptamers to recognize 100 ng of HIV-1 recombinant IN in sandwich ELONA are shown in **Figure 5A****.** None combination detected any of the two negative controls (NC1: 200 ng BSA protein and NC2 PBS/MgCl₂ without recombinant protein). HIV-1 IN was detected by all aptamer combinations with Apt2-HCINp1 or Apt3-HCINp1 as capture aptamer (C4: Apt2-HCINp1/Apt1-HCINp1, C5: Apt2-HCINp1/Apt2-HCINp1, C6: Apt2-HCINp1/Apt3-HCINp1, C7: Apt3-HCINp1/Apt1-HCINp1, C8: Apt3-HCINp1/Apt2-HCINp1, C9: Apt3-HCINp1/Apt3-HCINp1). None of the three remaining combinations containing the Apt1 - HCINp1 capture aptamer (C1 to C3) detected recombinant HIV-1 integrase. This may be due to Apt1-HCINp1 lower stability, possibly caused by its poorer predictions of G-quadruplex formation compared to Apt2-HCINp1 and Apt3-HCINp1.

The aptamer combination C5 (Apt2-HCINp1/Apt2-HCINp1) showed the highest affinity to HIV-1 IN. Further analysis showed that C5 detected IN significantly better than C4 (p = 0.0049), C6 (p = 0.0045), C7 (p = 0.0014), C8 (p = 0.0082) and C9 (p = 0.0011) **(****Figure 5A****).** This superiority could be attributed to the use of Apt2-HCINp1 aptamer as both capture and detection aptamer in the sandwich ELONA, benefiting from: 1) its highest affinity towards integrase as indicated by a lower K_{D} (30.95 ± 4.79 nM); 2) the highest max G-score, suggesting a better prediction of G-quadruplex formation and, consequently, higher stability and 3) that HIV IN is a homodimer, favouring the capture of detection of each monomer by the same aptamer.

Sensitivity of the optimal aptamer combination C5 was assesed by ELONA sandwich in streptavidin-coated 96-well plates with recombinant HIV-1 IN ranging from 3.13 to 100 ng, also testing 200 ng BSA used as a negative control (NC1). C5 detected recombinant HIV-1 IN in a concentration dependent manner up to 25 ng (p = 0.0119) (estimated to be to 9.13·10⁸ HIV virions/well or VL 9.26 log copies HIV-1 RNA/well, corresponding to 7.27·10⁴ pg of P24 capsid protein) **(****Figure 5B****).**

### Detection of HIV in culture supernatants, sera and plasma using the Apt2-HCINp1 aptamers pair in a sandwich ELONA

To confirm HIV detection by the C5 aptamer combination (Apt2-HCINp1/Apt2-HCINp1) we tested different HIV-infected samples types in sandwich ELONA: HIV-1 cell-free viral supernatant cultures, sera, and plasma. All twelve cell-free supernatants **(****Figure 6A****)** with VL ranging 8 to 10 log cp/mL, and 3 of the 4 sera **(****Figure 6B****)** with VL ranging 4.8 to 6.1 log cp/mL were successfully detected by the sandwich ELONA assay, showing a significant differences compared to their respective HIV negative controls (see **Figure 6** legend). However, none of the two plasma samples (around 5 log VL) yielded a positive result **(****Figure 6C****).** The sensitivity of the sandwich ELONA for HIV IN detection in clinical samples (detecting samples with at least 4.81 log cp/ml, 3.23·10³ virions/well, 8.84·10⁻⁵ ng IN/well, corresponding to 2.57·10⁻¹ pg P24) was approximately five logs more sensitive than previously estimated when testing recombinant IN protein (9.26 log copies HIV-1 RNA/well, 9.13·10⁸ virions/well, corresponding to 7.27·10⁴ pg P24). Regarding HIV-1 variants, the six HIV-1 group M variants (subtypes A, B, C and D, and CRF01_AE and CRF02_AG) infecting the supernatant panel were detected, which cause a highly significant proportion of HIV infections. As a result, Apt2-HCINp1 would be able to detect a significant range of current HIV infections.

The aptamer combination C5 did not cross-react with hepatitis C virus (HCV) after testing a HCV-positive serum containing 2.2·10⁷ HCV virions/ml in sandwich ELONA **(****Figure 7****).**

Despite the ELONA assay's complexity, time consumption, and limited sensitivity, it is valuable for characterizing aptamers. However, since anti-HIV aptamers have demonstrated the ability to detect HIV proteins across the most prevalent variants, it is essential to develop new molecular diagnostic POC test based on highly sensitive nanotechnologies and anti-HIV aptamers These new tests are described herein and are aim to achieve sensitivities superior to those of PCR and fourth- and fifth-generation immunoassays.

### Example 2

### MATERIAL AND METHODS

### Selection of highly conserved and exposed peptides in HIV PR among the HIV variants.

To identify highly conserved peptides in the PR of HIV-1 and HIV-2 variants, we first selected the most conserved peptides within the PR based on previous studies. Then, in November 2022, we downloaded one sequence per patient from all HIV-1 and HIV-2 PR sequences available at Los Alamos National Laboratory HIV Sequence Database (LANL). This database included types, groups, subtypes, sub-subtypes, and CRFs. URFs were not included in the study. Sequences containing stop codons and/or interrogation marks were eliminated, and the remaining sequences were aligned by Muscle v3.8.31. Subsequently, we analysed the presence of the previously identified highly conserved peptides across all HIV variants, using an in-house bioinformatics tool (EpiMolBio) designed and used in our Laboratory for genetic variability analysis. EpiMolBio has been programmed in JAVA OpenJDK version 18.0.2-ea using Apache NetBeans IDE 14 and is a freely available software designed for investigating the genetic variability of clinically significant viruses, microorganisms, genes, and proteins of biological or biomedical interest for epidemiological, diagnostic, or therapeutic purposes. Its user-friendly interface makes it accessible to users with limited bioinformatics expertise and provides an intuitive platform for genetic analysis and exploration. After the conservation study to identify the percentage of sequences with the peptides totally conserved per HIV variant, we explored the peptides exposure in the PR with both PyMOL Molecular Graphics System v2.5.2 program (Schrödinger, LLC) and Cn3D. For that, we used the three-dimensional (3D) HIV-1 and HIV-2 PR structures from RCSB Protein Data Bank: 2P3A and 3S45, respectively.

### Aptamers selection by Systematic Evolution of Ligands by Exponential Enrichment (SELEX)

Synthetic random single stranded DNA (ssDNA) population (P3-RND40) and non-labelled and labelled primers for aptamer's selection were obtained from IBA GmbH (Göttingen, Germany). All the aptamers were synthetized by Biomers GmbH (Göttingen, Germany). PR peptides were synthetized by ProteoGenix (Schiltigheim, France) and recombinant HIV-1 PR (ARP-11781) was provided by BEI Resources previously known as HIV Reagent Program.

Aptamers were selected by Systematic Evolution of Ligands by Exponential Enrichment (SELEX). For that, we performed iterative rounds of SELEX method of ssDNA P3-RND40 library. These oligonucleotides contained a central randomized region of 40 nucleotides flanked by two conserved regions of 18 nucleotides in each end (5'- 128 GCGGATGAAGACTGGTGT(N)40GCCCTAAATACGAGCAAC -3'). P3-RND40 was denatured 10 min at 90 °C and then cooled for another 10 min on ice. For the initial SELEX round, 2 nmol of ssDNA were mixed with 2 µg of the corresponding recombinant peptide in 200 µL of selection buffer (20 mM Tris-HCl, pH 7.4, 1 mM MgCl₂, 150 mM NaCl, 5 mM KCl, 0.2% bovine serum albumin or BSA) and incubated for 30 min at 37 °C. The bound aptamer-peptide complexes were purified by adding 20 µL of High Capacity NeutrAvidin Agarose resin (Pierce, 134 ThermoFisher) at 4 °C for 1 h for capture the peptide. After washing three times with 1 mL of selection buffer, the aptamer-peptide complexes were re-suspended in 20 µL of distilled H2O and amplified by PCR using the primers named F3 (5'-GCGGATGAAGACTGGTGT-3') and R3 137 (5'-GTTGCTCGTATTTAGGGC-3') under the conditions of 1 µM/primer, 250 µM dNTPs, in a final volume of 200 µL at 56 °C for 25 cycles. PCR product was ethanol precipitated. After round six (R6), the aptamer pool was amplified and labelled by PCR using digoxigenin-labelled primer F3 and non-labelled primer R3.

### Analysis of aptamer-HCPRp2 complexes by qPCR

To analyse the aptamers pool in the different rounds of the SELEX we performed a combined strategy dependent on quantitative polymerase chain reaction (qPCR) amplification curve and melting curve analysis. First, we incubated 1 µg of the biotinylated HCPRp2 peptide with 15 µL of High Capacity NeutrAvidin Agarose resin (Pierce, 147 ThermoFisher) at 4 °C on a shaker overnight. The rounds R3, R5, R6, and the initial population P3-RND40 (0.1 µg/mL) were incubated with resin-HCPRp2 complexes for 30 min at 37 °C with stirring. After centrifugation at 12,000 g for 10 min, complexes were washed four times with 1 mL of selection buffer, and finally, the resin was suspended in 20 µL of H2O and incubated at 90 °C for 10 min. Quantitative analysis was performed by qPCR using Quantimix Easy kit (Biotools, Spain) and F3 and R3 oligonucleotides, following the manufacturer's instructions in an iQ5, equipment (Bio-Rad, Barcelona, Spain). For the analysis of the individual aptamers obtained from round R6, each clone (0.1 µg/mL) were incubated with resin-HCPRp2 complexes as above. In parallel, the same amount of each clone was incubated with 15 µL of NeutrAvidin Agarose resin without HCPRp2. Aptamer cloning and sequencing Round six (R6) of the aptamer SELEX was cloned using TA Cloning^{™} Kit with pCR^{™}2.1 Vector (Invitrogen) following manufacturer's instructions. Individual clones were sequenced by Sanger method M13 Forward (GTAAAACGACGGCCAG) and M13 Reverse 162 (CAGGAAACAGCTATGAC) primers (IBA GmbH, Germany).

### Bioinformatic analysis of anti-HIV-1 protease aptamers

For aptamer secondary structure prediction, selected ssDNA sequences were introduced in the DNA Folding Form application at 25 °C and 37 °C in 150 mM [Na+] and 1 mM [Mg⁺²]. The DNA Folding results of the secondary ssDNA aptamer structures were used to model their 3D structures by the RNAcomposer server previously converting the ssDNA aptamer sequences into RNA. Then, the generated 3D RNA models were transformed back into DNA using the x3DNA server which mutates Uracils in Thymines and replaces ribose sugar with deoxyribose, generating a new output pdb file. We also predicted the presence of G-quadruplexes in the ssDNA aptamer sequences with QGRS Mapper, compared with an in-house bioinformatics tool (EpiMolBio Apt), since that structure increases stability and nuclease resistance.

### Indirect ELONA to analyse aptamers' affinity, specificity and sensitivity to recombinant PR

The Enzyme-Linked-Oligonucleotide-Assay (ELONA) is a technique similar to ELISA (Enzyme Linked Immunosorbent Assay) that uses oligonucleotides such as aptamers rather than antibodies as the recognition elements. ELONA provides a robust platform for the aptamer screening and characterization (affinity, sensitivity, specificity...), which can later be integrated into advanced diagnostic or therapeutic applications. In indirect ELONA, the target molecule (in our case, the HIV Protease) is immobilized on the surface of the well, and detection is achieved using a digoxigenin-labelled aptamer (detection aptamer). To analyse the aptamers' affinity for the HIV-1 recombinant PR, the protein was diluted to 1 ng/µL concentration (100 ng/well) and incubated overnight at 4 °C on a shaking platform. Selected digoxigenin-labelled aptamers were diluted in PBS/MgCl₂ [1 mM] at different concentrations (6.25-800 nM). To evaluate the aptamers' specificity against other HIV recombinant proteins from BEI Resources (retrotranscriptase, ARP-3555; integrase, ARP-9420; P24, ARP-13126; and Gp41, ARP-12027) we incubated 100 µL (100 ng/well) of each recombinant protein overnight at 4 °C on a shaking platform and the concentration of the digoxigenin-labelled aptamers were five times their K_{D}. Similar experiments were performed coating the wells with different concentrations of the HIV1 recombinant PR (0.03-1 ng/µL; 3.13-100 ng/well) to assess the aptamers' sensitivity at five times their K_{D} concentration.

### Analysis of the best combination of aptamers to increase sensitivity.

For identifying the best combination of the selected aptamers and the best conditions to increase their sensitivity, we performed different sandwich ELONA experiments. In sandwich ELONA, two aptamers (capture, detection) are used to "sandwich" the target molecule. Capture biotin-labelled or not-labelled aptamers were diluted to 0.5 µM in PBS/MgCl₂ [1 mM] and 100 µL were incubated overnight at 4 °C in 96-well microtiter plates coated with and without streptavidin (NUNC, ThermoFisher Scientific), respectively, and washed three times with PBS/Tween 0.1%. We add 200 µL of 5% BSA in PBS/MgCl₂ [1 mM] for blocking well sites without capture aptamer. To explore the sensitivity of the best combination of aptamers, HIV1 recombinant PR was diluted at different concentrations (0.01-0.125 ng/µL; 0.78-12.5 ng/well) and 100 µL of each concentration were add to different wells. Detection digoxigenin-labelled aptamers were diluted in PBS/MgCl₂ [1 mM] at five times their K_{D} concentration and then denatured for 10 min at 95 °C and cooled for 10 min on ice. Next, 100 µL of digoxigenin labelled aptamers were added to each well, and anti-digoxigenin-POD or anti-digoxigenin poly-POD antibody diluted 1:1,000 with 0.2% BSA in PBS/MgCl₂ [1 mM] were added and developed using ABTS solution as above.

### Clinical samples

Finally, we used the best combination of the selected aptamers to detect different clinical samples. We used four cell-free supernatants from BEI Resources being two HIV-1 group M subtype B (ARP-317 and ARP-11252, GenBank accession n°: M17449 and AY713408, respectively), one HIV-2 group A (ARP-600, GenBank accession n°: AY965906), and one HIV-2 group B (GenBank accession n° not available). Moreover, we used one plasma carrying HIV group M CRF02_AG recombinant from Equatorial Guinea with 6.1 log of Viral Load (VL) (GenBank accession n°: OL470763), and two viral samples (Genbank accession n°: KC473827 and JX140658) expanded and diluted in defribrinated plasma at approximately 10⁴ RNA-HIV1 copies/mL (4 log VL) carrying HIV-1 group M subtype B from the EQAPOL Genetic Diversity Panel (Duke Human Vaccine Institute. For this purpose, a sandwich ELONA was conducted on 96-well microtiter plate with streptavidin by incubating 0.5 µM of the biotin-labelled capture aptamer in PBS/MgCl₂ [1 mM] overnight with agitation. The following day, the samples were lysed for 30 minutes with lysis buffer (Triton [60 mM] and Tris-HCl [0.6%]) at ratio 2:1. In plasma samples, after the lysis, we dissociated antigen-antibody complexes with dissociation buffer (Glycine-HCl [0.2 M]) at 1:1 ratio of lysed samples. Subsequently, the pH was neutralized using NaOH [1 M] to restore the pH to physiological conditions. During sample pre-treatment, the plate was washed three times with PBS/Tween 0.1%, and the wells were blocked with 200 µL of 5% BSA diluted in PBS/MgCl₂ [1 mM]. Subsequently, 100 µL of the pre-treated samples were incubated in different wells, and a negative HIV dried blood sample (DBS) was used as a negative control (NC). Detection digoxigenin-labelled aptamers were diluted in PBS/MgCl₂ [1 mM] at five times their K_{D} concentration. Then, they were denatured for 10 min at 95 °C and cooled for 10 min on ice. Next, 100 µL of digoxigenin-labelled aptamers were added to each well, and anti-digoxigenin-poly-POD antibody diluted 1:1,000 with 0.2% BSA in PBS/MgCl₂ [1 mM] was added and developed using ABTS solution as above.

### Aptamer truncation

Three modified versions of each selected anti-PR aptamer were designed based on the secondary structure predicted by the DNA Folding Form application and the possible presence of predicted G-quadruplex using the QGRS mapper program. The aptamers were synthesized and conjugated with digoxigenin, and their affinity for the HIV-1 recombinant PR was analysed by indirect ELONA using at first only two concentrations of detection aptamers, [200 nM] or [400 nM], and then as previously described with the best ones.

### Statistical analysis

Data are presented as an average value ± standard error of the mean (SEM) from three to nine independent measurements in separate experiments, and analysed using GraphPad Prism v8.0 (San Diego, CA). For the affinity study, data were analysed using nonlinear regression responding to a one-site binding curve with an equation y = (x · Bₘₐₓ)/(x + K_{D}), where Bₘₐₓ is the maximal binding and K_{D} is the concentration of ligand required to reach half-maximal binding. The statistical significance was performed by analysis of variance ANOVA followed by Dunnet's test or unpaired t test with Welch's correction when we cannot assume equal variances. These tests were run depending on the experiment and what we want to compare. Significance was assumed at p<0.05.

### Results

The following peptides and anti-PR aptamers were used:
HCPRp1: **LLDTGADD** (8aa)
HCPRp2 **GGIGGFI** (7 aa)
AptGI6.1F = Apt1-HCPRp2 (SEQ ID NO 8): 5'-GCGGATGAAGACTGGTGTAGTCCGGCGGGTTGGTTGGGTCGGGTTTTCTTTTTT TGGTGCCCTAAATACGAGCAAC-3'
AptG16.16F = Apt2-HCPRp2 (SEQ ID NO 9):5'-GCGGATGAAGACTGGTGTGCAGTTGGCGTTGTGTTTGTCCGTGTGTTTCTTGGC GTTTGCCCTAAATACGAGCAAC-3'

### Selection of highly-conserved and exposed PR peptide for aptamers selection

Previous published studies reported the amino acid conservation rates along HIV-1 and HIV-2 PR, and we explored the most conserved epitopes with EpiMolBio tool. Then, we identified two highly conserved PR peptides: HCPRp1, (8 aa), and HCPRp2 (7 aa). To explore the conservation level of the selected peptides across HIV variants at study time, we downloaded 6,436 HIV-1/HIV-2 PR sequences of 141/8 variants from LANL, eliminating the sequences with stop codons and interrogation marks using the conservation tool of the EpiMolBio software. Then, we quantified the percentage of sequences in each HIV variant with HCPRp1 and HCPRp2 sequences 100% conserved **(****Figure 8A****)** after analysing 217,066/1,445 HIV-1/HIV-2 PR sequences from 140/5 variants and 219,465/1,538 HIV-1/HIV-2 PR sequences from 140/5 variants for each PR peptide, respectively. Both peptides were present (100% conserved) in up to 90% of HIV-1 groups and HIV-2 variants sequences (98.0% sequences for HCPRp1 / 95.9% sequences for HCPRp2), except in HIV-2 group A, where HCPRp2 was found in 88.6% sequences. To explore the peptides exposure in the PR, we visualized 3D HIV-1 and HIV-2 PR structures showing that HCPRp1 was hidden in the HIV-1 and HIV-2 PR dimers while HCPRp2 was exposed **(****Figure 8B****).** Therefore, we selected HCPRp2 as a highly-conserved and exposed PR peptide, being the best candidate and target for the aptamer selection by SELEX.

### Selection of two aptamers targeting HCPRp2 peptide

The ssDNA aptamers specific for HCPRp2 were selected from P3-RND40 library by SELEX. As an alternative method to ELONA to see the evolution of the Apt-HCPRp2 population, a qPCR assay was performed to analyse the Apt-HCPRp2 enrichment after six selection rounds. The results showed an increase in the Apt-HCPRp2 signal obtained after SELEX rounds R5 and R6 relative to the initial P3-RND40 population (2.5-fold and 4-fold, respectively), and between rounds R5 and R6 (1.6-fold). There was a drop in fluorescence in qPCR after testing the initial rounds due to the great variability in sequences, which decreased as the selection progressed, leading to an increasing enrichment of the target; this caused fluorescence to increase, which indicates that selection was successful. The same occurred with the melting curve of these assays, where there was an evolution toward higher Tm and higher fluorescence peaks for successive rounds with less sequence variability. The assay evolution indicated a decrease in sequence variability with each progressive round of selection, thus resulting in an enrichment of aptamers against the HCPRp2 peptide. We analysed different Apt-HCPRp2 characteristics to determine whether this aptamer population could be used in an HIV detection system for HIV diagnostic purpose. After the previous results, we proceeded with the cloning of the round R6 Apt-HCPRp2 population to isolate and characterize individual aptamers, as described in Materials and Methods. We identified and selected two aptamers for that goal: Apt1-HCPRp2 and Apt2-HCPRp2.

### Apt1-HCPRp2 and Apt2-HCPRp2 aptamers characterization

*Bioinformatics analyses.* The sequences of Apt1-HCPRp2 and Apt2-HCPRp2 were analysed by three different bioinformatics programs: QGRS mapper, DNA folding, and EpiMolBio Apt. DNA-folding studied the potential most stable secondary structures for each aptamer in the selection conditions reported in Materials and Methods. Although Apt2-HCPRp2 would be more stable than Apt1-HCPRp2 at 25 °C and 37 °C, considering its lower predicted Gibbs free energy value, only Apt1-HCPRp2 had pairs of guanines capable of constructing G-quadruplex predicted by QGRS mapper and EpiMolBio Apt results. Moreover, the aptamers showed different secondary structures **(****Figure 9****),** suggesting that each of them could bind each HIV-1 PR with different affinity. Representative 3D structures for the aptamers, predicted from their respective secondary structure information, are shown too in **Figure 9****.**

*Affinity.* To study the affinity of both aptamers for HIV-1 PR, we performed an indirect ELONA assay in which the 96-well plates were coated with HIV-1 recombinant PR, and different concentrations (6.25-800 nM) of digoxigenin-labelled aptamers were tested as described in Materials and Methods. The results shown that Apt1-HCPRp2 can detect the HIV-1 recombinant PR with K_{D} = 10.04 ± 4.66 nM and Apt2- HCPRp2 with K_{D} = 15.43 ± 4.86 nM **(****Figure 10A****).**

*Sensitivity.* To determine the sensitivity of the two ssDNA aptamers we performed an indirect ELONA in which 96-well plates were coated with quantities ranging from 3.13 to 100 ng of HIV-1 recombinant PR or 200 ng of BSA protein as NC and incubated with the digoxigenin-labelled aptamer, as described in Materials and Methods. The sensitivity curve **(****Figure 10B****)** showed that Apt1-HCPRp2 could detect the HIV-1 recombinant PR in a concentration-dependent manner until 6.25 ng of protein vs. BSA with significant differences (p = 0.0378), while Apt2-HCPRp2 could detect until 12.5 ng of protein vs. BSA (p = 0.0359).

*Specificity.* The results of the specificity ELONA shown that both ssDNA aptamers were able to detect the recombinant HIV PR with significant differences. **(****Figure 10C****).** Apt1- HCPRp2 and Apt2-HCPRp2 could also detect the recombinant HIV IN with significant differences vs. BSA and PBS/MgCl₂, while they could not recognize the rest of the tested HIV recombinant proteins.

*Aptamers best combinations.* To determine the best combinations of the two selected aptamers, we performed a sandwich ELONA in which 96-well plates were coated with 0.5 µM of the capture aptamer, 100 ng of HIV-1 recombinant PR or 200 ng of BSA protein as NC and then incubated with the detection digoxigenin-labelled aptamers, as described in Materials and Methods. The results shown that the four possible aptamer combinations detected the HIV-1 recombinant PR in a significant manner vs. BSA and other NC without detection aptamer. Between the four combinations (C1: Apt1-HCPRp2 / Apt1-HCPRp2; C2: Apt1-HCPRp2 / Apt2-HCPRp2; C3: Apt2-HCPRp2 / Apt1-HCPRp2; C4: Apt2-HCPRp2 / Apt2- HCPRp2) only C1 was able to detect the HIV-1 recombinant PR significantly vs. C2 (p = 0.0415). C1 and C3 were the best combinations, but C1 provided the highest absorbance **(****Figure 11A****).**

*Strategies to increase the HIV-1 recombinant protease recognition in sandwich ELONA Aptamer combination sensitivity.* We performed different sandwich ELONA to increase the HIV-1 recombinant PR detection by our selected aptamers. After choosing C1 and C3 as the better combinations of aptamers in the experiments done before, we executed the sandwich ELONA using 96-well microtiter plates coated with or without streptavidin and anti-digoxigenin-horseradish peroxidase (HRP) or anti-digoxigenin-poly-HRP as a secondary detection antibody as described in Materials and Methods. The results shown that using 96-well microtiter plates coated with streptavidin vs. without streptavidin increased the significant differences of the HIV-1 recombinant PR detection by C1 and C3 vs. BSA. Furthermore, when we used anti-digoxigenin-poly-HRP as secondary detection antibody instead of anti-digoxigenin-HRP, C1 and C3 were able to detect 3.13 ng/well of HIV-1 recombinant PR (estimated 3.76·10⁸ HIV virions/well) with significant differences vs. BSA (p = 0.0002 and p = 0.0197, respectively) **(****Figure 11B****).**

### Aptamer truncation.

We analysed three truncated aptamers modified from Apt1-HCPRp2 and Apt2-HCPRp2 **(****Figure 12A****).** This truncated aptamers exhibited lower affinity compared to the parental ones **(****Figure 12B****).** Nevertheless, we selected aptamer Apt2-HCPRp2-t1 for further affinity characterization **(****Figure 12C****).** After performing an affinity indirect ELONA assay with 96- well plates coated with HIV-1 recombinant PR and different concentrations (50-3,200 nM) of the digoxigenin-labelled aptamer Apt2-HCPRp2-t1, the results shown that Apt2-HCPRp2-t1 had a higher K_{D}, which meant less affinity than its parental aptamer Apt2-HCPRp2. This demonstrate that our parental aptamers were the best options, with no need of truncation to improve their affinity for the HIV-1 recombinant PR.

### Detection of HIV protease in culture supernatants by Apt1-HCPRp2 pair in sandwich ELONA

We performed a sandwich ELONA with one of the most sensitive capture/detection aptamer combinations (C1, see **Figure 11A****)** to confirm if the best PR specific aptamer combination could detect different types of clinical samples. We tested HIV-1 or HIV-2 cell-free virus in supernatant culture, plasma from an HIV infected person from Equatorial Guinea, and viral culture supernatants diluted in plasma from the EQAPOL Genetic Diversity Panel, as described in Materials and Methods. The EQAPOL Genetic Diversity Panel samples (M6, M7) with an estimated VL of 5.13·10³ -7.33·10³ were not detected, as well as the plasma sample (M5) with an estimated 4.61·10⁵ of VL, due to the detection limit of the aptamers combination (C1) used (3.13 ng PR/well, corresponding to 3.76·10⁸ virions/well assuming 250 PR/virion). However, the remaining samples with VL between 4.17·10⁸ -6.91·10⁹ were detected in the sandwich ELONA, with significate differences between the negative control (M1, p = 0.0284; M2, p = 0.0010; M3, 427 p = 0.0173; M4, p = 0.0407) (**Figure 13**).

### DISCUSSION OF RESULTS EXAMPLE 2.

HIV and acquired immunodeficiency syndrome (AIDS) remains a non-cure disease, and is still infecting new people each year, with more that 1.3 million new infections in 2023, despite the actual diagnosis methods and treatment protocols. In adults, an early HIV diagnosis is essential, since the per-person probability of transmitting HIV-1 is most closely correlated with the viral burden in blood. The risk of contagion from patients with acute, early infection appears to be much higher than that from patients with established infection. Regarding the paediatric population, without early HIV diagnosis and treatment, more than half of vertically-infected children born to HIV-infected women will die by age two. This is why reducing the window period of HIV diagnosis is a priority. It will permit infected people to know their status and treat them earlier preventing the infection of new people. However, the actual methods of diagnosis have a window period of 10 days if they are NAATs or around 18-14 days if they are fourth or fifth generation ELISA assays, respectively. These techniques are expensive to use in low-income countries with limited infrastructures and trained personnel. It includes Eastern and Southern Africa, with 20.8 million people living with HIV (53% of all people living with HIV in the world) and 500,000 new HIV infections per year. In this example we explored new molecular diagnostic strategies focused on detecting HIV proteins using specific ssDNA aptamers, which share similar properties than antibodies but offer greater affordability and cost-effectiveness in synthesis and production. Unlike antibodies, aptamers can target both immunogenic and non-immunogenic epitopes, broadening their applicability. Aptamers have also shown potential in various fields, including disease diagnosis, therapeutic development, food safety, biosensing, toxin detection, drug delivery, and nanoparticle labelling. Their use in virology is increasing, and recent advancements have extended their application to HIV research, primarily for therapeutic purposes and targeting non-translated regulatory regions, and viral proteins, such as Gp120, Tat, P24, RT, and PR. Despite significant potential of aptamers for both treatment and diagnostic applications, the HIV PR has been relatively underexplored, with only one study to date focusing on the development of anti-PR aptamers to inhibit HIV replication *in vitro.* In that study, the researchers obtained anti-PR aptamers with K_{D} ranging from 2 to 22 nM, which is comparable to the K_{D} values of our selected anti-PR aptamers, further supporting the promise of our approach.

It is crucial that the identified HIV targets in diagnostic tests (viral genome regions targeted by primers/probes used in diagnostic PCR and immunogenic viral epitopes recognized by antibodies in serological assays) remain as conserved as possible among viral variants in order to detect the majority of virus strains. To evaluate the highest conservation level of the PR peptide recognized by our selected ssDNA aptamers targeting HIV PR, we examined all available PR sequences (217,066 from HIV-1 and 1,445 from HIV-2) attributed to over 140 HIV variants (types, subtypes, sub-subtypes, and CRFs) from the LANL database at the time of the study. These sequences represent the majority of the genetic variability of HIV circulating worldwide. The EpiMolBio program (https://www.epimolbio.com), developed in the laboratory and previously used for genetic variability analysis and protein conservation in HIV and in the new SARS-CoV-2 virus, the causative agent of the COVID-19 pandemic, was employed for this purpose. The obtained results demonstrated that the conservation of HCPRp1 and HCPRp2 peptides was very high, being completely conserved in 98.0% and 95.9% of analysed HIV variants, respectively. Only the HCPRp2 peptide was exposed in the 3D PR structure, being considered the best target for designing aptamers capable of recognizing the HIV PR protein.

We have identified the first two ssDNA aptamers selected against a highly-conserved and exposed HIV-1 PR peptide. These aptamers showed high affinity and sensitivity in binding to HIV-1 recombinant PR in ELONA, the gold standard assay to characterize aptamers. They could also recognize HIV-1 recombinant IN, but at a significant lower level. The observed cross-reactivity of this aptamer's pair might not pose a problem for distinguishing HIV-positive samples from non-HIV samples, as HIV-1 PR and IN are not encoded by the human genome. All Apt-HCPRp2 combinations were able to detect HIV recombinant PR with significant differences being Apt1-HCPRp2 + Apt1-HCPRp2 (C1), the one with more absorbance. The variations in sensitivity of the two described apatmers directed to the same exposed and highly conserved peptide can be attributed to several factors inherent to the aptamers themselves. Each aptamer has distinct dissociation constants (K_{D}), sensitivities, and three-dimensional structures, all of which influence its interaction with the HIV PR. when aptamers are used individually or in combination.

We also showed that the best strategy providing better sensitivity detecting low ng/well of HIV recombinant PR in sandwich ELONA was using 96-well microtiter plates with streptavidin and the secondary anti-digoxigenin antibody poly-HRP. For further ELONA studies, we should need to analyse cross-reactivity with other viruses' proteins and human PR to ensure the robustness of these aptamers for diagnostic purposes. It is also expected that these aptamers recognize PR of different HIV-1 and HIV-2 variants carrying the peptide used for the aptamers' selection. This will be further studied in optimized ELONA, testing more infected culture supernatants with high VL and samples from the EQAPOL panel carrying different HIV variants.

ELONAs are complex assays, take long time, are not very sensitive (since they detect ng of viral PR, estimated 3.76·10⁸ HIV virions/well), and in some cases, they are highly dependent on assay variables and reagents affecting reproducibility. However, ELONAs are often employed in the initial screening of aptamers since they are easy to perform and optimize in the laboratory, facilitating the characterization of aptamers in terms of affinity, sensitivity, and specificity. Additionally, they allow the evaluation of different aptamers combinations in a sandwich ELONA using capture-detection aptamer pairs. This can be particularly useful before transitioning to other new ultrasensitive nanotechnologies or biosensors under development for diagnosis utilizing aptamers. ELONAs generally show comparable or even superior detection limits to traditional ELISA (generally picograms), depending on the target and the amplification strategies used. In our experiments, ELONA assays did not provide high sensitivity when were performed following the enzyme-based procedure described in Methods, detecting the target (HIV-1 PR) at nanogram levels. However, the integration

## Claims

1. An aptamer capable of binding the integrase (IN) of HIV-1 and HIV-2 variants or portions thereof.

2. The aptamer according to claim 1, wherein the aptamer is capable of binding a protein selected from any one of the list consisting of: HCINp1 (11 aa) (SEQ ID NO 4, PYNPQSQGWE), HCINp2 (9 aa) (SEQ ID NO 5, NFKRKGGIG), HCINp3 (9 aa) (SEQ ID NO 6, LLWKGEGAV), HCINp4 (8 aa) (SEQ ID NO 7, IKVVPRRK) and/or HCINp5 (10aa) (SEQ ID NO 15, LLWKGEGAVV).

3. The aptamer according to claim 1, wherein the aptamer is capable of binding HCINp1 (11 aa) (SEQ ID NO 4).

4. The aptamer according to claim 3, wherein the aptamer is selected from the list consisting of SEQ ID NO 1 to 3 or SEQ ID NO 16 to 18, or an aptamer sharing at least about 95%, at least about 95.5%, at least about 96%, at least about 96.1 %, at least about 96.2%, at least about 96.3%, at least about 96.4%, at least about 96.5%, at least about 96.6%, at least about 96.7%, at least about 96.8%, at least about 96.9%, at least about 97%, at least about 97.1 %, at least about 97.2%, at least about 97.3%, at least about 97.4%, at least about 97.5%, at least about 97.6%, at least about 97.7%, at least about 97.8%, at least about 97.9%, at least about 98%, at least about 98.1 %, at least about 98.2%, at least about 98.3%, at least about 98.4%, at least about 98.5%, at least about 98.6%, at least about 98.7%, at least about 98.8%, at least about 98.9% or at least about 99% sequence identity/homology with any of the sequences set forth in SEQ ID NO 1 to 3 or SEQ ID NO 16 to 18.

5. The aptamer according to claim 3, wherein the aptamer is set forth in SEQ ID NO 2, or an aptamer sharing at least about 95%, at least about 95.5%, at least about 96%, at least about 96.1 %, at least about 96.2%, at least about 96.3%, at least about 96.4%, at least about 96.5%, at least about 96.6%, at least about 96.7%, at least about 96.8%, at least about 96.9%, at least about 97%, at least about 97.1 %, at least about 97.2%, at least about 97.3%, at least about 97.4%, at least about 97.5%, at least about 97.6%, at least about 97.7%, at least about 97.8%, at least about 97.9%, at least about 98%, at least about 98.1 %, at least about 98.2%, at least about 98.3%, at least about 98.4%, at least about 98.5%, at least about 98.6%, at least about 98.7%, at least about 98.8%, at least about 98.9% or at least about 99% sequence identity/homology with the sequence set forth in SEQ ID NO 2.

6. The aptamer according to claim 3, wherein the aptamer is set forth in SEQ ID NO 2.

7. The aptamer according to claim 3, wherein the aptamer is a mixture comprising at least two, at least three or at least four of the aptamers according to any one of the preceding claims.

8. The aptamer according to any one of the preceding claims, wherein the aptamer comprises a DNA-based aptamer.

9. A method of identifying a HIV infection in a subject, the method comprising: contacting a sample of the subject with the aptamer or mixture therefrom according to any one of claims 1-8; and detecting a binding event at the aptamer(s).

10. The method according to claim 9, wherein the method is a method of identifying a current HIV infection in the subject, and a binding event at any of the aptamer(s) is indicative of a current HIV infection in the subject, optionally wherein the bound aptamer is specific to single HIV serotype and the binding event is indicative of a current HIV infection of said serotype in the subject.

11. The method according to claim 9 or claim 10, wherein the method comprises a competitive binding assay method.

12. The method according to claim 9 or claim 10, wherein the method is in the form of a "sandwich" assay, where a first binder or capturing agent (e.g. an aptamer) serves to capture a target analyte (e.g. a HIV protein) and a second binder or detector agent (e.g. a further reporter aptamer) is used to detect the captured target analyte.

13. The method according to claim 12, wherein the assay is selected from the list consisting of enzyme immunoassays (EIA) / enzyme-linked immunosorbent assay (ELISA), enzyme-linked aptamers assays (ELAA) /enzyme-linked oligonucleotide assay (ELONA), radioimmunoassay RIA, strip assays, lateral flow assays (LFA), bio-layer interferometry (BLI), the detection through Surface plasmon resonance (SPR), colorimetric changes using gold nanoparticle aggregations, voltammetric, and electrochemical techniques and the like.

14. The method according to any one of claims 9-13, wherein the method is carried out within one week following the initial infection of the subject.

15. A kit for identifying a HIV infection in a subject, the kit comprising an aptamer according to any one of claims 1-6 or the mixture of aptamers according to claim 8.
